# EUROPÄISCHE PATENTSCHRIFT

(11) **EP 1 608 645 B1**
(45) Veröffentlichungstag und Bekanntmachung des Hinweises auf die Patenterteilung: **02.05.2007**
(21) Anmeldenummer: 04718641.6
(22) Anmeldetag: 09.03.2004
(51) Int. Cl.: C07D 413/12, C07D 401/12, C07D 453/06, C07D 403/12, C07C 275/26, A61K 31/5377, A61K 31/4155, A61K 31/4748, A61P 7/02

(54) **PYRAZOLIDIN-1,2-DICARBONSÄURE-1-((PHENYL)-AMID)-2-((PHENYL)-AMID) DERIVATE ALS KOAGULATIONSFAKTOR XA INHIBITOREN ZUR BEHANDLUNG VON THROMBOSEN**
PYRAZOLIDINE-1,2-DICARBOXYLDIPHENYLAMIDE DERIVATIVES AS COAGULATION FACTOR XA INHIBITORS FOR THE TREATMENT OF THROMBOSES
DERIVES DE L'ACIDE PYRAZOLIDINE-1,2-DICARBOXYLIQUE-1-(PHENYLAMIDE)-2-(PHENYLAMIDE) COMME INHIBITEURS DU FACTEUR DE COAGULATION XA DANS LE TRAITEMENT DE THROMBOSES

(30) Priorität: 03.04.2003 DE 10315377; 30.06.2003 DE 10329295; 08.08.2003 DE 10336570
(43) Veröffentlichungstag der Anmeldung: 28.12.2005
(73) Patentinhaber: Merck Patent GmbH, 64293 Darmstadt (DE)
(72) Erfinder: MEDERSKI, Werner, 64673 Zwingenberg (DE); TSAKLAKIDIS, Christos, 69469 Weinheim (DE); DORSCH, Dieter, 64372 Ober-Ramstadt (DE); CEZANNE, Bertram, 64546 Mörfelden-Walldorf (DE); GLEITZ, Johannes, 64289 Darmstadt (DE)
(86) Internationale Anmeldenummer: PCT/EP2004/002407
(87) Internationale Veröffentlichungsnummer: WO 2004/087696

(56) Entgegenhaltungen:
- WO-A-01/64642
- WO-A-02/074735
- WO-A-03/024971
- DATABASE MEDLINE [Online] US NATIONAL LIBRARY OF MEDICINE (NLM), BETHESDA, MD, US; 1972 ROUBAL Z ET AL: "Newly synthesized pyrazolidine derivatives as potential activators of fibrinolysis and antiaggregating agents." Database accession no. NLM4669826 XP002283716 & ACTA UNIVERSITATIS CAROLINAE. MEDICA. MONOGRAPHIA. 1972, Bd. 52, 1972, Seiten 49-54, ISSN: 0567-8250
- DATABASE MEDLINE [Online] US NATIONAL LIBRARY OF MEDICINE (NLM), BETHESDA, MD, US; 1972 MURATOVÁ J ET AL: "Effect of pyrazolidine derivatives on experimental venous thrombosis in rabbits." Database accession no. NLM4273931 XP002283717 & ACTA UNIVERSITATIS CAROLINAE. MEDICA. MONOGRAPHIA. 1972, Bd. 52, 1972, Seiten 61-68, ISSN: 0567-8250
- DATABASE CAPLUS [Online] CHEMICAL ABSTRACTS SERVICE, COLUMBUS, OHIO, US; 1994 VAINIOTALO, P. ET AL: "N,N-Coupled heterobicycles from cyclic hydrazine derivatives. Part 6. Electron ionization mass spectrometry of some substituted 1-thiocarbamoyl and 1-carbamoylpyrazolidines" retrieved from STN Database accession no. 1994:190889 XP002283718 & JOURNAL OF HETEROCYCLIC CHEMISTRY ( 1993 ), 30(6), 1641-4 CODEN: JHTCAD; ISSN: 0022-152X, 1993,
- DATABASE CAPLUS [Online] CHEMICAL ABSTRACTS SERVICE, COLUMBUS, OHIO, US; 1973 ZINNER, G. ET AL: "Carbamoylation of hydrazine derivatives" retrieved from STN Database accession no. 1973:97543 XP002283719 & ARCHIV DER PHARMAZIE (WEINHEIM, GERMANY) ( 1973 ), 306(1), 35-44 CODEN: ARPMAS; ISSN: 0365-6233, 1973,
- DATABASE CAPLUS [Online] CHEMICAL ABSTRACTS SERVICE, COLUMBUS, OHIO, US; 1972 BOLLBUCK, GUENTER ET AL: "Reactions of 4-aryl semicarbazides" retrieved from STN Database accession no. 1972:59168 XP002283720 & JOURNAL FUER PRAKTISCHE CHEMIE (LEIPZIG) ( 1971 ), 313(4), 773-7 CODEN: JPCEAO; ISSN: 0021-8383, 1971,

## Beschreibung

Die Erfindung betrifft Verbindungen der Formel I 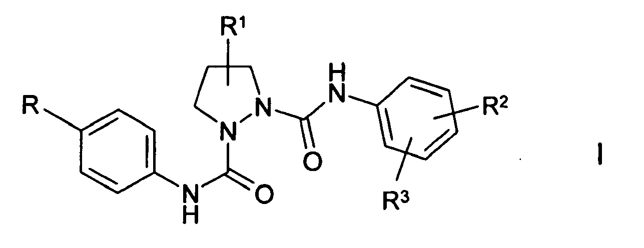 worin
- R: Hal oder -C≡C-H,
- R¹: H, =O, OH, OA, A-COO-, Ph-(CH₂)ₙ-COO-, Cycloalkyl-(CH₂)ₙ-COO-,
- Ph: unsubstituiertes Phenyl,
- R²: H, Hal oder A,
- R³: 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
- A: unverzweigtes, verzweigtes oder cylisches Alkyl mit 1-10 C-Atomen, worin auch 1-7 H-Atome durch F und/oder Chlor ersetzt sein können,
- Hal: F, Cl, Br oder I,
- n: 0, 1, 2, 3 oder 4,
bedeuten,
sowie ihre pharmazeutisch verwendbaren Derivate, Salze, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor Vlla, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben.

Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.
Andere Carbonsäureamidderivate sind aus WO 02/48099 und WO 02/57236 bekannt, andere Pyrrolidinderivate sind in WO 02/100830 beschrieben.

Andere Faktor Xa - Inhibitoren kennt man aus WO 01/64642 A und aus WO 02/074735 A.
Roubal Z. et al. beschreiben andere Pyrazolidinderivate als Fibrinolyseaktivatoren mit antiaggregierenden Eigenschaften: DATABASE MEDLINE [Online] US NATIONAL LIBRARY OF MEDICINE (NLM), BETHESDA, MD, US; 1972; XP002283716 Database accession no. NLM4669826.
Muratovä J. et al. beschreiben Wirkungen von Pyrazolidinderivaten auf Thrombosen: DATABASE MEDLINE [Online] US NATIONAL LIBRARY OF MEDICINE (NLM), BETHESDA, MD, US; 1972;
XP002283717 Database accession no. NLM4273931.
P. Vainiotalo et al. beschreiben in J. Het. Chem. 30, 1641 (1993) substituierte 1-Thiocarbamoyl- und 1-Carbamoylpyrazolidine.
G. Zinner et al. beschreiben in Archiv der Pharmazie 306, 35-44 (1973) die Herstellung von anderen Pyrazolidinen.
Wiederum andere Pyrazolidine sind von G. Bollbuck et al. in Journal für Praktische Chemie 313, 773-777 (1971) beschrieben.
In WO 03/024971 A1 sind 6,7-Dihydro-5H-pyrazolo[1,2-a]pyrazol-1-one offenbart.

Weitere heterocyclische Derivate kennt man aus der WO 03/045912.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor Vlla, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in *Circulation* **1996**, *94*, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in *Thrombosis and Haemostasis* **1990**, *63*, 220-223 beschrieben.

Die Messung der Inhibierung von Faktor Xa kann z.B. nach der Methode von T. Hara et al. in *Thromb. Haemostas.* **1994**, *71,* 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in *Thrombosis Research* **1996**, *84*, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J.

Chang et al. in *Journal of Biological Chemistry* **1998,** *273,* 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor Vlla und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.

Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo*, oder als Antikoagulantien zur Konservierung von Blut, Plasma und anderen Blutprodukten *in vitro*. Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.

Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-2 sowie ihrer pharmazeutisch verwendbaren Derivate, Solvate und Stereoisomere, dadurch gekennzeichnet, daß man
a) eine Verbindung der Formel II 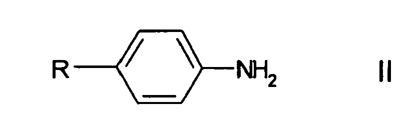 worin R die in Anspruch 1 angegebene Bedeutung hat,
   mit einem Chloroformiatderivat zu einem intermediären Carbamatderivat umsetzt,
   das anschließend mit einer Verbindung der Formel III-1 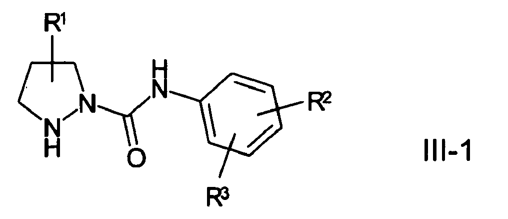 worin
   R¹, R² und R³ die in Anspruch 1 angegebene Bedeutung haben,
   und falls R¹ OH bedeutet, die OH-Gruppe gegebenenfalls geschützt vorliegt,
   umgesetzt wird,
   und anschließend gegebenenfalls die OH-Schutzgruppe abgespalten wird,
   oder
b) eine Verbindung der Formel IV 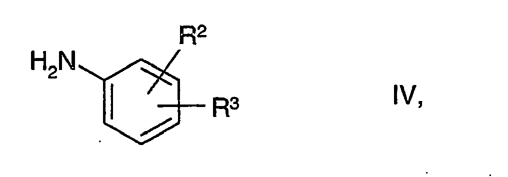
worin R² und R³ die in Anspruch 1 angegebene Bedeutung haben, mit einem Chloroformiatderivat zu einem intermediären Carbamatderivat umsetzt,
das anschließend mit einer Verbindung der Formel III-2 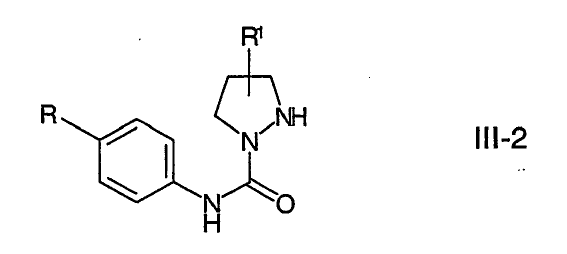 worin R und R¹ die in Anspruch 1 angegebene Bedeutung haben, und falls R¹ OH bedeutet, die OH-Gruppe gegebenenfalls geschützt vorliegt,
umgesetzt wird,
und anschließend gegebenenfalls die OH-Schutzgruppe abgespalten wird,
und/oder
eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter R, R² und R³ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.
Cycloalkyl bedeutet vorzugsweise Cyclopropyl, Cyclobutyl, Cylopentyl, Cyclohexyl oder Cycloheptyl.

R bedeutet vorzugsweise Hal oder -C≡C-H.

R¹ bedeutet vorzugsweise H, =O, OH, OA, A-COO-, Ph-(CH₂)ₙ-COO- oder Cycloalkyl-(CH₂)ₙ-COO-, besonders bevorzugt H, =O oder OH.

R² bedeutet vorzugsweise H, Cl, F oder Alkyl mit 1-6 C-Atomen, wie z.B. Methyl, Ethyl, Propyl, Butyl oder Trifluormethyl.

R³ bedeutet ganz besonders bevorzugt 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H-*pyridin-1-y1, 2-Oxo-1*H*-Pyrazin-1-yl, 2-Oxo-imidazolidin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4H-[1,4]Oxazin-4-yl.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II, III-1, III-2 und IV sind teilweise neu.

Verbindungen der Formel I können vorzugsweise erhalten werden, indem man Verbindungen der Formel II bzw. IV mit einem Chloroformiatderivat, z.B. 4-Nitrophenylchlorformiat zu einem intermediären Carbamat umsetzt und anschließend mit Verbindungen der Formel III-1 bzw. III-2 umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Phenolkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können ferner erhalten werden, indem man aus Verbindungen der Formel I, die eine OH- oder NH-Schutzgruppe enthalten, die Schutzgruppe abspaltet.

Bevorzugte Ausgangsstoffe für die Schutzgruppenabspaltung sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -OH oder -COOH eine Gruppe -OR" oder -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen oder Alkylsilylschutzgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind. Ganz besonders bevorzugt ist, falls R¹ OH bedeutet die *tert*.-Butyldimethylsilyl-Schutzgruppe.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°. Die Abspaltung von Alkylsilylschutzgruppen, wie z.B.der *tert*.-Butyldimethylsilyl-Schutzgruppe, gelingt z.B. mit Tetra-n-butylammoniumfluorid.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Die bevorzugten Bedeutungen der Reste in den nachfolgend aufgeführten Verbindungen der Formel III-1, III-2 und VI entsprechen denen, wie oben für die Verbindungen der Formel I aufgeführt, falls nicht ausdrücklich etwas anderes angegeben ist.

Gegenstand der Erfindung sind auch die Zwischenverbindungen der Formel III-1 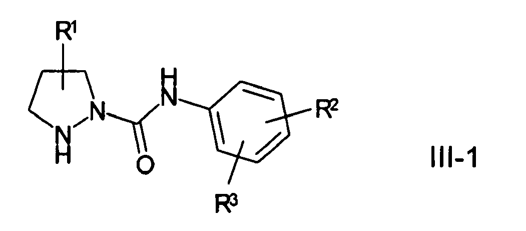 worin
- R¹: H, =O, OR⁶, OA, A-COO-, Ph-(CH₂)ₙ-COO- oder Cycloalkyl-(CH₂)ₙ-COO-,
- Ph: unsubstituiertes Phenyl,
- R²: H, Hal oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2,6-Dioxo-piperidin1-yl, 2-Oxo-piperazin-1-yl, 2,6-Dioxopiperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
- R⁶: eine OH-Schutzgruppe,
- A: unverzweigtes, verzweigtes oder cylisches Alkyl mit 1-10 C-Atomen, worin auch 1-7 H-Atome durch F und/oder Chlor ersetzt sein können,
- Hal: F, Cl, Br oder I,
- n: 0, 1, 2, 3 oder 4,
bedeuten,
sowie deren Isomere und Salze.

Gegenstand der Erfindung sind besonders bevorzugt die Zwischenverbindungen der Formel III-1, worin
- R¹: H, =O oder OR⁶,
- R²: H, Hal oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl, 4*H*-[1,4]Oxazin-4-yl,
- R⁶: eine Alkylsilyl-Schutzgruppe,
- A: unverzweigtes, verzweigtes oder cylisches Alkyl mit 1-10 C-Atomen, worin auch 1-7 H-Atome durch F und/oder Chlor ersetzt sein können,
- Hal: F, Cl, Br oder I,
- n: 0, 1, 2, 3 oder 4,
bedeuten,
sowie deren Isomere und Salze.

Gegenstand der Erfindung sind auch die Zwischenverbindungen der Formel III-2 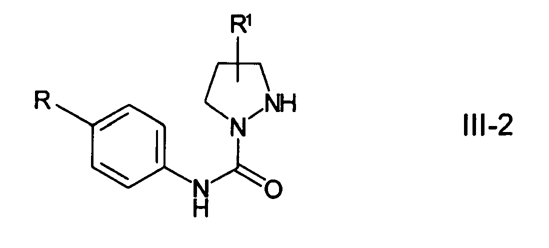 worin
- R: Hal oder -C≡C-H,
- R¹: H, =O oder OR⁶,
- R⁶: eine Alkylsilyl-Schutzgruppe,
- Hal: F, Cl, Br oder I,
bedeuten,
wobei, falls R¹ H bedeutet, R nicht Cl bedeutet,
sowie deren Isomere und Salze.

Die Verbindung N-(4-Chlorphenyl)-1-pyrazolidincarboxamid ist beschrieben in J. Heterocyclic Chem. (1993), 30(6), 1641-4 und in Pharmazie (1991), 46(6), 418-22.

Vorstufen zur Herstellung der Verbindungen der Formel III-1 bzw. III-2 sind die Verbindungen der Formel VI 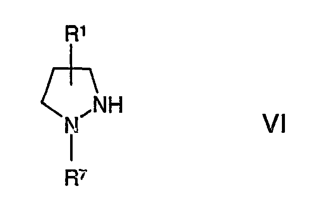 worin
- R¹: die bei den Formeln III-1 bzw. III-2 angegebenen Bedeutungen hat und
- R⁷: eine Aminoschutzgruppe bedeutet.
Vorzugsweise bedeutet die Aminoschutzgruppe *tert*.-Butyloxycarbonyl (BOC) oder Benzyloxycarbonyl (Z).

Die Herstellung von Verbindungen der Formel VI, worin R¹ H bedeutet, z.B. der Pyrazolidin-Verbindungen der Formel VI-1 und VI-2, erfolgt nach einer bekannten Herstellvorschrift ausgehend von Z-geschütztem *tert*.-Butylcarbazan und 1,3-Dibrompropan in 2 Stufen ( Dutta, A. S.; Morley, J. S. *J. Chem. Soc. Perkin Trans 1* **1975,** 1712)

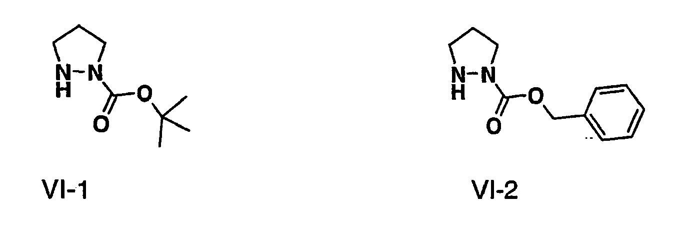

Verbindungen der Formel VI, worin
- R¹: OH oder OR⁶,
- R⁶: eine Silyl-Schutzgruppe,
- R⁷: BOC oder Z
bedeuten, sind neu.

Gegenstand der Erfindung sind daher auch die Zwischenverbindungen der Formel VI 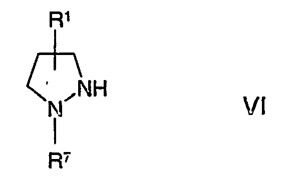 worin
- R¹: OH oder OR⁶,
- R⁶: eine Silyl-Schutzgruppe,
- R⁷: *tert*.-Butyloxycarbonyl (BOC) oder Benzyloxycarbonyl (Z)
bedeuten,
sowie deren Isomere.

Besonders bevorzugt sind Verbindungen der Formel VI, worin R⁶ *tert*.-Butyldimethylsilyl bedeutet.

Die Verbindungen werden analog nachstehendem Schema direkt aus *tert*.-Butylcarbazan und silylgeschützem 1,3-Dibrom-propan-2-ol in einer Stufe gewonnen. Die Reaktion gelingt nicht mit der freien OH-Verbindung. 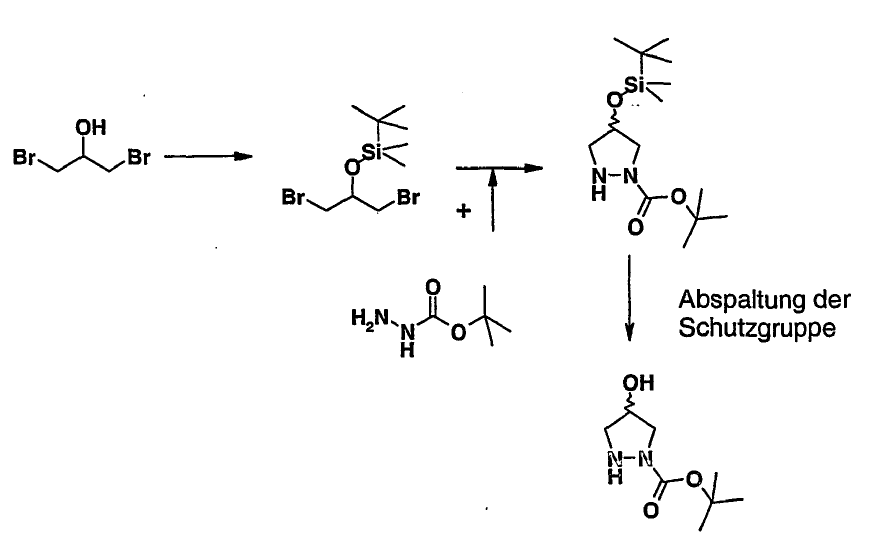 Die Abspaltung der Silylschutzgruppe erfolgt, falls erforderlich, durch bekannte Methoden, z.B. mit Tetra-n-butylammoniumfluorid.

Gegenstand der Erfindung ist weiterhin ein Verfahren zur Herstellung von Verbindungen der Formel VI 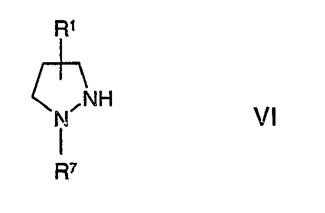 worin
- R¹: OH oder OR⁶,
- R⁶: eine Silylschutzgruppe,
- R⁷: *tert*.-Butyloxycarbonyl (BOC) oder Benzyloxycarbonyl (Z)
bedeuten,
sowie deren Isomere,
erhältlich durch Umsetzung einer Verbindung der Formel VII

R⁷-NHNH₂ VII,

worin R⁷ BOC oder Z bedeutet,
mit silylgeschütztem 1,3-Dibrom-propan-2-ol,
und gegebenenfalls, anschließender Abspaltung der Schutzgruppe.

Besonders bevorzugt ist ein Verfahren zur Herstellung von Verbindungen der Formel VI, worin
R⁶ *tert*.-Butyldimethylsilyl bedeutet.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Salze, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung ist ferner die Verwendung von Verbindungen der Formel I und/oder ihrer pharmazeutisch verwendbaren Derivate, Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
Massenspektrometrie (MS): EI (Elektronenstoß-Ionisation) M⁺
ESI (Electrospray lonization) (M+H)⁺ (wenn nichts anderes angegeben)

### Beispiel 1

### Pyrazolidin-1,2-dicarbonsäure-1-[(4-ethinyl-phenyl)-amid]-2-{[3-chlor-4-(3-oxo-morpholin-4-yl)-phenyl]-amid} ("A1")

### Stufe 1

### 2-[3-Chlor-4-(3-oxo-morpholin-4-yl)-phenylcarbamoyl]-pyrazolidin-1-carbonsäure-tert.-butylester:

340 mg (1,5 mmol) 4-(4-Amino-2-chlor-phenyl)-morpholin-3-on werden in 10 mL Dichlormethan (DCM) gelöst, anschließend nacheinander 302 mg (1,5 mmol) 4-Nitrophenylchloroformiat und 121 µL (1,5 mmol) Pyridin zugegeben und 1 h bei RT gerührt. Zu dieser Reaktionsmischung werden 300,0 mg (0,7 mmol) Pyrazolidin-1-carbonsäure-*tert*.-butylester und 0,765 mg (4,5 mmol) *N*-Ethyldiisopropylamin gegeben. Nach 20 h Rühren bei RT wird wie üblich aufgearbeitet. So erhält man 420 mg (57,3%) Stufe 1; MS
(FAB) m/z = 425/427 (M+H)⁺.

### Stufe 2

### Pyrazolidin-1-carbonsäure-[3-chlor-4-(3-oxo-morpholin-4-yl)-phenyl]-amid:

410 mg (0,84 mmol) Stufe 1 werden in 5 mL Doxan gelöst und mit 5 ml (20 mmol) 4M HCl in Dioxan versetzt. Nach 5 h Rühren bei RT wird wie üblich aufgearbeitet und man erhält so 330 mg (Y = 91,5%) Stufe 2. MS (FAB)
m/z = 325/327 (M+H)⁺.

### Stufe 3

84,3 mg (0,7 mmol) 4-Ethinylanilin werden in 10 mL DCM gelöst, anschließend nacheinander 140,7 mg (0,7 mmol) 4-Nitrophenylchloroformiat und 56,4 µL (0,7 mmol) Pyridin zugegeben und 1 h bei RT gerührt. Zu dieser Reaktionsmischung werden 300,0 mg (0,7 mmol) Stufe 2 und 0,475 mL (2,8 mmol) *N*-Ethyldiisopropylamin gegeben. Nach 20 h Rühren bei RT wird wie üblich aufgearbeitet. Der Rückstand wird aus EE umkristallisiert und so erhält man 170 mg (Y = 50,6%) "A1" als Kristalle; MS (FAB) m/z = 468/470 (M+H)⁺.

Analog erhält man die nachstehenden Verbindungen
Pyrazolidin-1,2-dicarbonsäure-1-[(4-chlorphenyl)-amid]-2-{[3-chlor-4-(3-oxo-morpholin-4-yl)-phenyl]-amid} ("A2"), M+H⁺ 478, 480;
Pyrazolidin-1,2-dicarbonsäure-1-[(4-chlorphenyl)-amid]-2-{[4-(2-oxo-2*H*-pyridin-1-yl)-phenyl]-amid}, M+H⁺ 438, 440;
Pyrazolidin-1,2-dicarbonsäure-1-[(4-chlorphenyl)-amid]-2-{[3-methyl-4-(3-oxo-morpholin-4-yl)-phenyl]-amid}, M+H⁺ 458, 460;
Pyrazolidin-1,2-dicarbonsäure-1-[(4-chlorphenyl)-amid]-2-{[3-fluor-4-(3-oxo-morpholin-4-yl)-phenyl]-amid}, M+H⁺ 462, 464;
Fyrazolidin-1,2-dicarbonsäure-1-[(4-chlorphenyl)-amid]-2-{[3-chlor-4-(2-oxo-2*H*-pyridin-1-yl)-phenyl]-amid}, M+H⁺ 472, 474;
Pyrazolidin-1,2-dicarbonsäure-1-[(4-chlorphenyl)-amid]-2-{[3-chlor-4-(2-aza-bicyclo[2.2.2]-octan-3-on-2-yl)-phenyl]-amid}, M+H⁺ 502, 504;
Pyrazolidin-1,2-dicarbonsäure-1-[(4-chlorphenyl)-amid]-2-{[3-methyl-4-(2-oxo-pyrrolidin-yl)-phenyl]-amid},
4-Oxo-pyrazolidin-1,2-dicarbonsäure-1-[(4-chlorphenyl)-amid]-2-{[4-(3-oxo-morpholin-4-yl)-phenyl]-amid},
Pyrazolidin-1,2-dicarbonsäure-1-[(4-chlorphenyl)-amid]-2-{[4-(2-oxo-piperidin-yl)-phenyl]-amid}, M+H⁺ 442, 444;
Pyrazolidin-1,2-dicarbonsäure-1-[(4-chlorphenyl)-amid]-2-{[4-(3-oxo-morpholin-4-yl)-phenyl]-amid}, M+H⁺ 444, 446;
Pyrazolidin-1,2-dicarbonsäure-1-[(4-chlorphenyl)-amid]-2-{[2-fluor-4-(3-oxo-morpholin-4-yl)-phenyl]-amid}, M+H⁺ 462, 464;
Pyrazolidin-1,2-dicarbonsäure-1-[(4-chlorphenyl)-amid]-2-{[3-trifluormethyl-4-(2-aza-bicyclo[2.2.2]-octan-3-on-2-yl)-phenyl]-amid}, M+H⁺ 536, 538;
Pyrazolidin-1,2-dicarbonsäure-1-[(4-chlorphenyl)-amid]-2-{[4-(2-aza-bicyclo[2.2.2]-octan-3-on-2-yl)-phenyl]-amid}, M+H⁺ 468, 470;
Pyrazolidin-1,2-dicarbonsäure-1-[(4-chlorphenyl)-amid]-2-{[4-(2-oxo-[1,3]oxazinan-3-yl)-phenyl]-amid}, M+H⁺ 444, 446;
Pyrazolidin-1,2-dicarbonsäure-1-[(4-ethinylphenyl)-amid]-2-{[4-(2-oxo-2*H*-pyridin-1-yl)-phenyl]-amid},
Pyrazolidin-1,2-dicarbonsäure-1-[(4-ethinyl-phenyl)-amid]-2-{[3-methyl-4-(3-oxo-morpholin-4-yl)-phenyl]-amid},
Pyrazolidin-1,2-dicarbonsäure-1-[(4-bromphenyl)-amid]-2-{[4-(2-oxo-2*H*-pyridin-1-yl)-phenyl]-amid} ("A3"), M+H⁺ 483.

### Beispiel 2

### 4-Hydroxy-pyrazolidin-1,2-dicarbonsäure-1-[(4-chlorphenyl)-amid]-2-{[4-(2-oxo-2H-pyridin-1-yl)-phenyl]-amid} ("B1")

### Stufe 1

### (2-Brom-1-brommethyl-ethoxy)-tert.-butyl-dimethyl-silan:

26,7 g (122,4 mmol) 1,3-Dibrom-propan-2-ol werden in 150 mL DCM gelöst und mit 18,35 g (269,59 mmol) Imidazol versetzt. Anschließend werden 18,47 g (122,54 mmol) *tert*.-Butyldimethylchlorsilan in 150 mL DCM zugetropft. Man lässt 18 h bei RT rühren, arbeitet wie üblich auf und erhält so als Rohprodukt 39,1 g (Y = 95,9%) Stufe 1 als farbloses Öl, welches direkt in Stufe 2 eingesetzt wird.

### Stufe 2

### 4-(tert.-Butyl-dimethyl-silanyloxy)-pyrazolidin-1-carbonsäure-tert.-butylester:

9,87 g (246,6 mmol) Natriumhydrid (60%ig) werden in 50 mL DMF suspendiert. Dann gibt man 15,5 g (117,4 mmol) *tert*.-Butylcarbazan in 100 mL DMF tropfenweise hinzu (starkes Schäumen). Nach 2 h Rühren bei RT werden nun 39,0 g (117,5 mmol) Stufe 1 hinzugetropft und 18 h bei RT gerührt. Man arbeitet wie üblich auf und erhält so 30,2 g (Y = 85%) Stufe 2 als braunes Rohöl, welches direkt in Stufe 3 eingesetzt wird.

### Stufe 3

### 4-(tert.-Butyl-dimethyl-silanyloxy)-2-(4-chlor-phenylcarbamoyl)-pyrazolidin-1-carbonsäure-tert.-butylester:

30,1 g (99,8 mmol) Stufe 2 werden in 200 mL DCM gelöst und mit 15,3 g (99,8 mmol) 4-Chlorphenylisocyanat versetzt und 5 h bei RT gerührt. Das Rohprodukt wird mit Wasser gewaschen, über Natriumsulfat getrocknet, vom Feststoff abfiltriert und zur trockne eingeengt. Nach Chromatographie an Kieselgel mit PE/EE = 8/2 erhält man so 23,4 g Stufe 3 (Y = 51%).

### Stufe 4

### 4-(tert.-Butyl-dimethyl-silanyloxy)-pyrazolidin-1-carbonsäure-(4-chlorphenyl)-amid:

Zu einer Lösung von 10,2 g (22,4 mmol) Stufe 3 in 50 mL DCM werden 25,0 mL (13,0 mmol) Trifluoressigsäure getropft und 1 h bei RT gerührt. Nach üblicher Aufarbeitung erhält man so 7,8 g (98%) Stufe 4; MS (FAB)
m/z = 356/358 (M+H)⁺.

### Stufe 5

### 4-(tert.-Butyl-dimethyl-silanyloxy)-pyrazolidin-1,2-dicarbonsäure-1-[(4-chlorphenyl)-amid]-2-{[4-(2-oxo-2H-pyridin-1-yl)-phenyl]-amide}:

145,3 mg (0,68 mmol) 1-(4-Amino-phenyl)-2*H*-pyridin-2-on werden in 2 mL DCM gelöst, anschließend nacheinander 157,2 mg (0,78 mmol) 4-Nitrophenylchloroformat und 62,96 µL (0,78 mmol) Pyridin zugegeben und 1 h bei RT gerührt. Zu dieser Reaktionsmischung werden 277,6 mg (0,78 mmol) Stufe 4, 0,4 mL (2,3 mmol) *N*-Ethyldiisopropylamin und 2 mL THF gegeben. Nach 18 h Rühren bei RT wird wie üblich aufgearbeitet und man erhält so 330 mg (Y = 74,5%) Stufe 5; MS (FAB) m/z = 568/570 (M+H)⁺.

### Stufe 6

### "B1":

330 mg (0,58 mmol) Stufe 5 werden in 4,0 mL THF gelöst und mit 250,0 mg (0,8 mmol) *Tetra*-*n*-butylammoniumfluorid unter Rühren bei RT versetzt und anschließend 1 h bei dieser Temperatur weitergerührt. Man verdünnt mit Wasser und gibt 1 N HCl-Lösung hinzu. Der ausgefallene Niederschlag wird abgesaugt, mit Wasser gewaschen und getrocknet. So erhält man 190 mg (Y = 72%) "B1" als Kristalle; MS (FAB) m/z = 454/456 (M+H)⁺.

Analog erhält man die nachstehenden Verbindungen
4-Hydroxy-pyrazolidin-1,2-dicarbonsäure-1-[(4-chlorphenyl)-amid]-2-{[4-(3-oxo-morpholin-4-yl)-phenyl]-amid}, M+H⁺ 460, 462;
4-Hydroxy-pyrazolidin-1,2-dicarbonsäure-1-[(4-chlorphenyl)-amid]-2-{[4-(2-oxo-piperidin-1-yl)-phenyl]-amid}, M+H⁺ 458, 460;
4-Hydroxy-pyrazolidin-1,2-dicarbonsäure-1-[(4-chlorphenyl)-amid]-2-{[3-methyl-4-(3-oxo-morpholin-4-yl)-phenyl]-amid}, M+H⁺ 474, 476;
4-Hydroxy-pyrazolidin-1,2-dicarbonsäure-1-[(4-chlorphenyl)-amid]-2-{[3-methyl-4-(2-oxo-pyrrolidin-yl)-phenyl]-amid}, M+H⁺ 458, 460;
4-Hydroxy-pyrazolidin-1,2-dicarbonsäure-1-[(4-chlorphenyl)-amid]-2-{[3-fluor-4-(3-oxo-morpholin-4-yl)-phenyl]-amid}, M+H⁺ 478, 480;
4-Hydroxy-pyrazolidin-1,2-dicarbonsäure-1-[(4-chlorphenyl)-amid]-2-{[3-chlor-4-(3-oxo-morpholin-4-yl)-phenyl]-amid}, M+H⁺ 494, 496;
4-Hydroxy-pyrazolidin-1,2-dicarbonsäure-1-[(4-chlorphenyl)-amid]-2-{[3-fluor-4-(2-oxo-pyrrolidin-yl)-phenyl]-amid}, M+H⁺ 478, 480;
4-Hydroxy-pyrazolidin-1,2-dicarbonsäure-1-[(4-chlorphenyl)-amid]-2-{[3-chlor-4-(2-oxo-2*H*-pyridin-1-yl)-phenyl]-amid}, M+H⁺ 488, 490;
4-Hydroxy-pyrazolidin-1,2-dicarbonsäure-1-[(4-chlorphenyl)-amid]-2-{[4-(2-aza-bicyclo[2.2.2]-octan-3-on-2-yl)-phenyl]-amid}, M+H⁺ 488, 486;
4-Hydroxy-pyrazolidin-1,2-dicarbonsäure-1-[(4-chlorphenyl)-amid]-2-{[3-trifluormethyl-4-(2-aza-bicyclo[2.2.2]-octan-3-on-2-yl)-phenyl]-amid}, M+H⁺ 552, 553;
4-Hydroxy-pyrazolidin-1,2-dicarbonsäure-1-[(4-chlorphenyl)-amid]-2-{[3-chlor-4-(2-aza-bicyclo[2.2.2]-octan-3-on-2-yl)-phenyl]-amid}, M+H⁺ 518, 520;
4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
(R)-4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
(R)-4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
(R)-4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
(S)-4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
(S)-4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
(S)-4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
4-Hydroxy-pyrazolidin-1,2-dicarbonsäure-1-[(4-bromphenyl)-amid]-2-{[4-(2-oxo-2*H*-pyridin-1-yl)-phenyl]-amid},
(S)-4-Hydroxy-pyrazolidin-1,2-dicarbonsäure-1-[(4-bromphenyl)-amid]-2-{[4-(2-oxo-2*H*-pyridin-1-yl)-phenyl]-amid},
(R)-4-Hydroxy-pyrazolidin-1,2-dicarbonsäure-1-[(4-bromphenyl)-amid]-2-{[4-(2-oxo-2*H*-pyridin-1-yl)-phenyl]-amid}.

### Beispiel 3

Durch Umsetzung von 1 Äquivalent der nachfolgend aufgeführten Hydroxyverbindungen
4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
4-Hydroxy-pyräzolidin-1,2-dicarbonsäure-1-[(4-ethinyl-phenyl)-amid]-2-{[4-(2-oxo-2*H*-pyridin-1-yl)-phenyl]-amid},
4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
mit 1,1 - 1,5 Äquivalenten der gewünschten Säurechloride in DCM bei Raumtemperatur erhält man nach üblicher Aufarbeitung die nachstehenden Verbindungen
4-Acetoxy-pyrazolidin-1,2-dicarbonsäure-1-[(4-ethinyl-phenyl)-amid]-2-{[3-chlor-4-(3-oxo-morpholin-4-yl)-phenyl]-amid},
4-Benzylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Benzoyloxy-pyrazolidin-1,2-dicarbonsäure-1-[(4-ethinyl-phenyl)-amid]-2-{[3-methyl-4-(3-oxo-morpholin-4-yl)-phenyl]-amid},
4-*tert*.-Butylcarbonyloxy-pyrazolidin-1,2-dicarbonsäure-1-[(4-ethinyl-phenyl)-amid]-2-{[3-chlor-4-(3-oxo-morpholin-4-yl)-phenyl]-amid},
4-Isobutylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Cyclohexylmethylcarbonyloxy-pyrazolidin-1,2-dicarbonsäure-1-[(4-ethinyl-phenyl)-amid]-2-{[3-methyl-4-(3-oxo-morpholin-4-yl)-phenyl]-amid},
4-Cyclopentylcarbonyloxy-pyrazolidin-1,2-dicarbonsäure-1-[(4-ethinyl-phenyl)-amid]-2-{[3-chlor-4-(3-oxo-morpholin-4-yl)-phenyl]-amid},
4-Cyclopropylmethylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Cyclobutylcarbonyloxy-pyrazolidin-1,2-dicarbonsäure-1-[(4-ethinyl-phenyl)-amid]-2-{[3-methyl-4-(3-oxo-morpholin-4-yl)-phenyl]-amid}.

### 14. Beispiele zur Herstellung von Zwischenverbindungen

### 14.1 Nach folgendem Schema lassen sich alle Verbindungen der folgenden Formel VI (mit R = H oder Methyl; n = 3, 4 oder 5) synthetisieren.

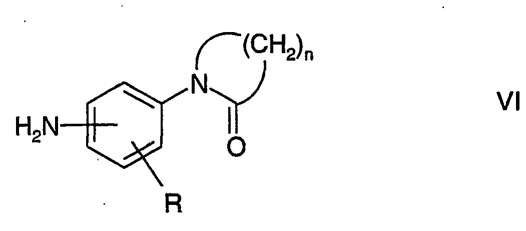

Z.B. Synthese von 1-(4-Amino-2-methylphenyl)-piperidin-2-on:

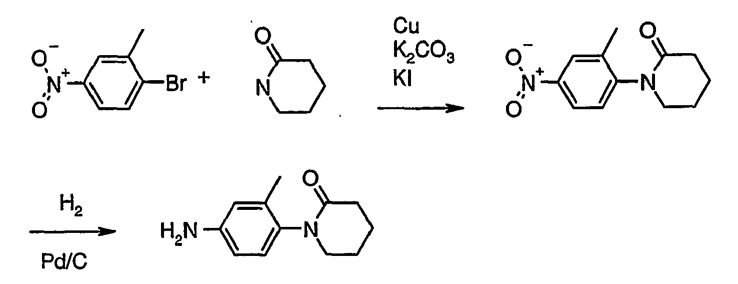

### 14.2 Synthese des Phenylpiperidonbausteins ohne Methylgruppe:

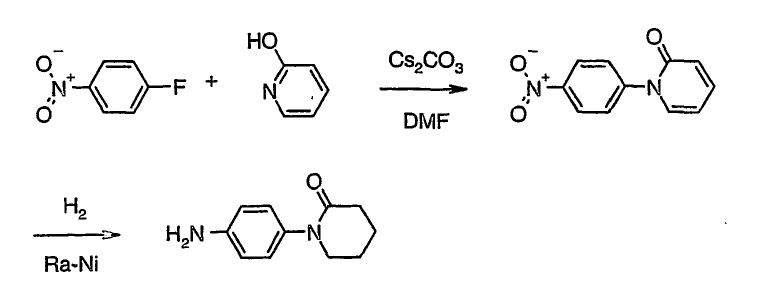

Die Herstellung von 1-(4-Amino-2-methyl-phenyl)-piperidin-2-on erfogt z.B. wie nachfolgend angegeben:

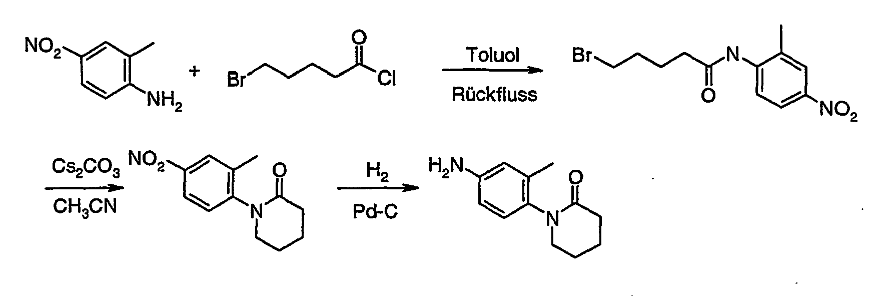

### 14.3 1-(4-Amino-phenyl)-1H-pyrazin-2-on

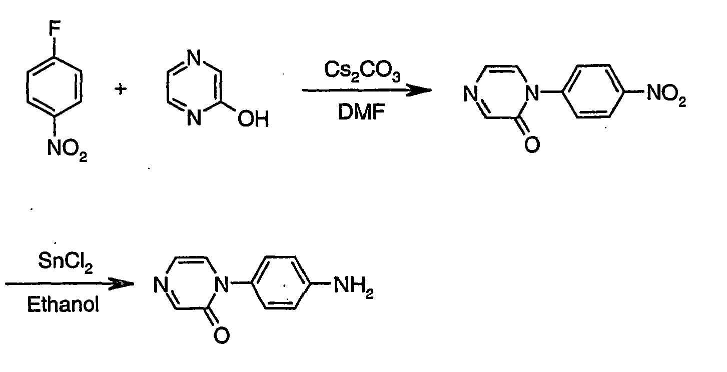

### 14.4 1-(4-Amino-2,5-dimethyl-phenyl)-piperidin-2-on

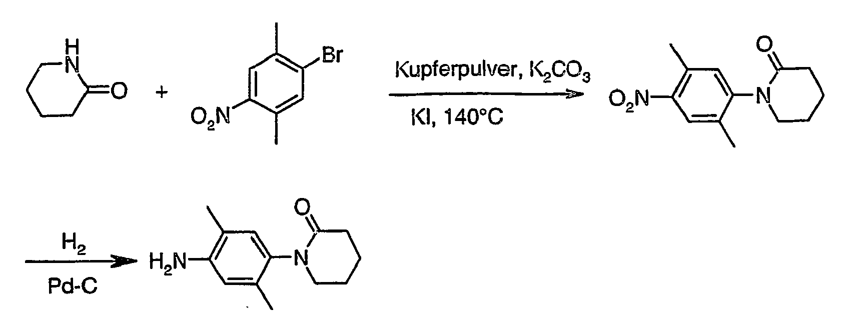

### 14.5 1-(4-Amino-3-methyl-phenyl)-piperidin-2-on

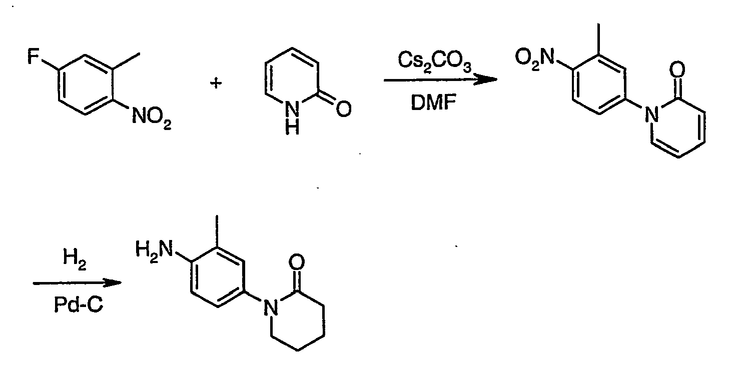

### 14.6 1-(5-Amino-pyridin-2-yl)-piperidin-2-on

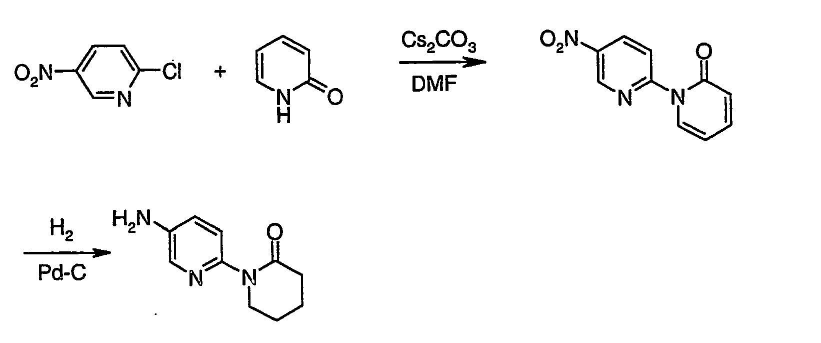

### 14.7 1-(4-Aminomethyl-phenyl)-piperidin-2-on

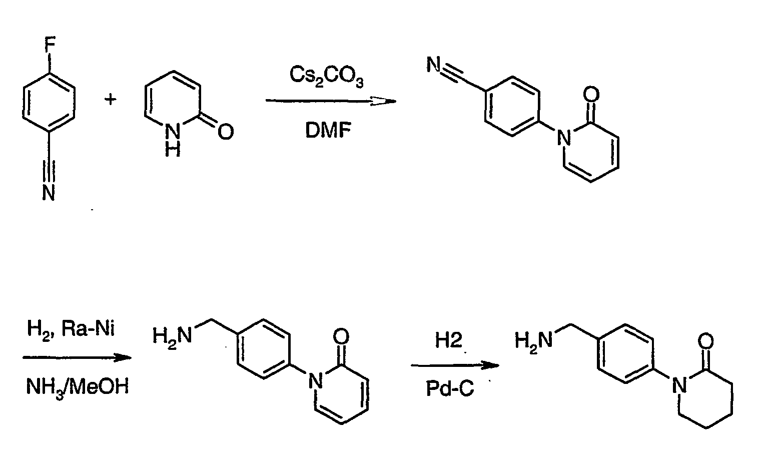

### 14.8 2-(4-Amino-phenyl)-2-aza-bicyclo[2.2.2]octan-3-on

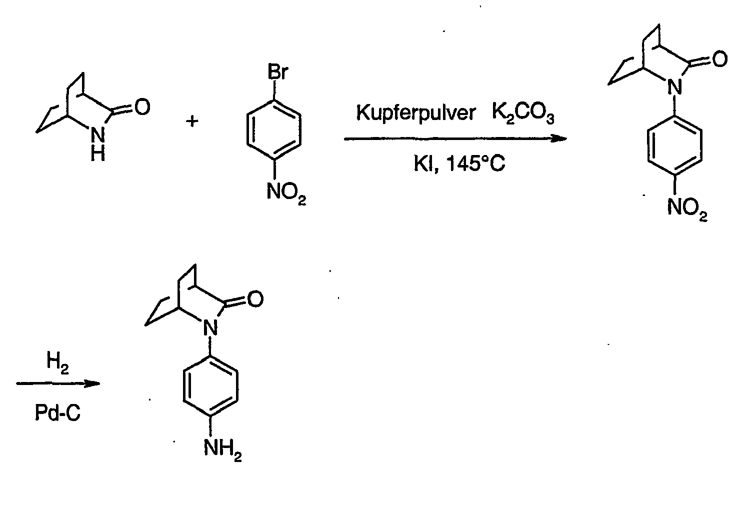

### 14.9 1-(3-Amino-6-ethyl-phenyl)-pyrrolidin-2-on

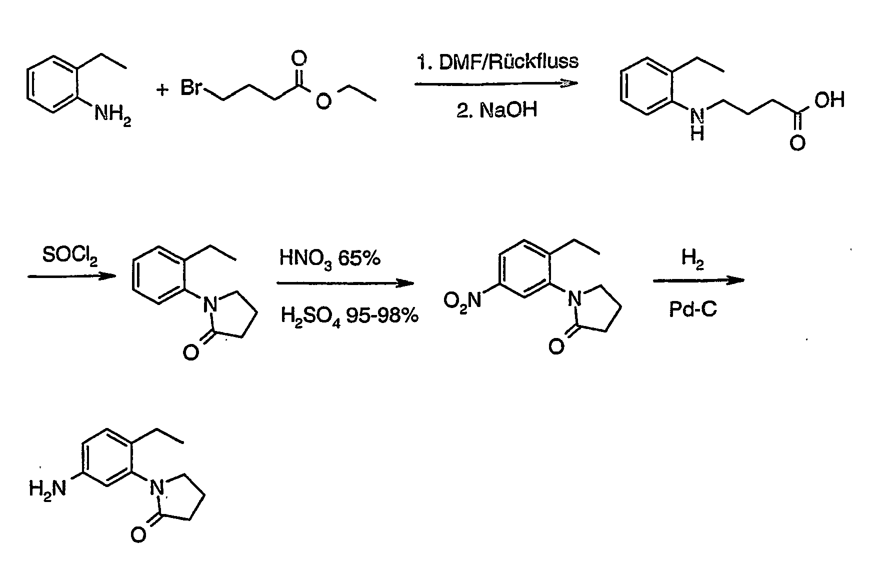

### 14.10 2-(4-Amino-2-trifluormethyl-phenyl)-2-aza-bicyclo[2.2.2]octan-3-on

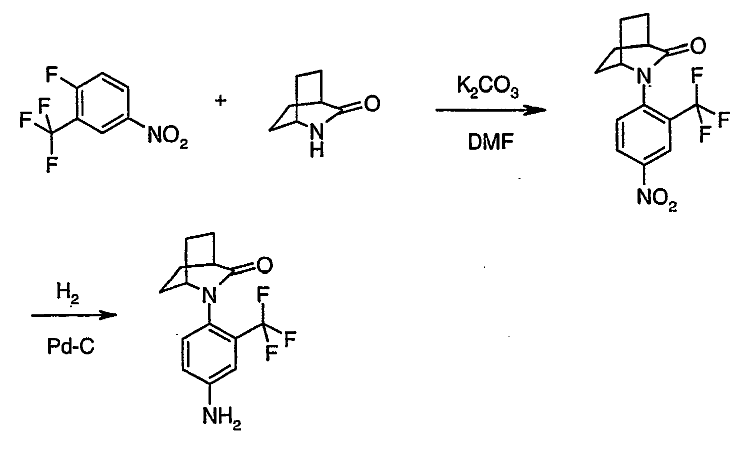

### 14.11 1-(4-Amino-3-chlor-phenyl)-pyrrolidin-2-on

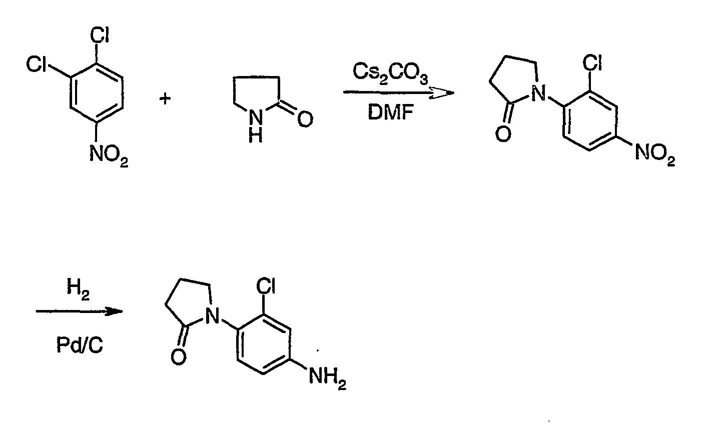

### 14.12 1-(4-Amino-2-trifluormethyl-phenyl)-piperidin-2-on

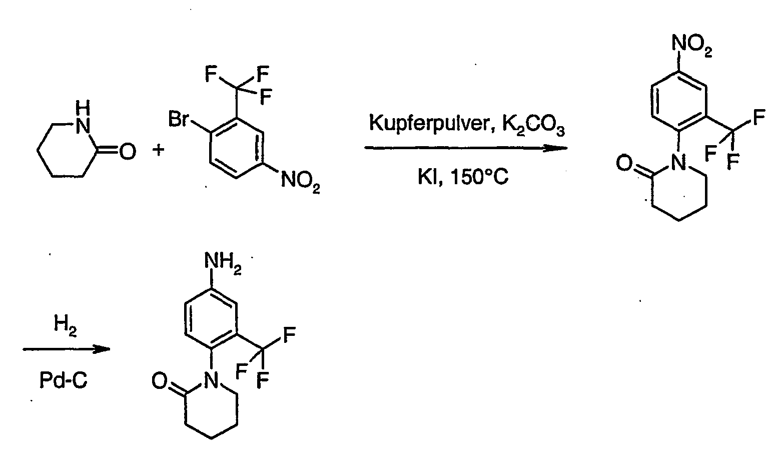

### 14.13 3-(4-Amino-2-methyl-phenyl)-[1,3]oxazinan-2-on

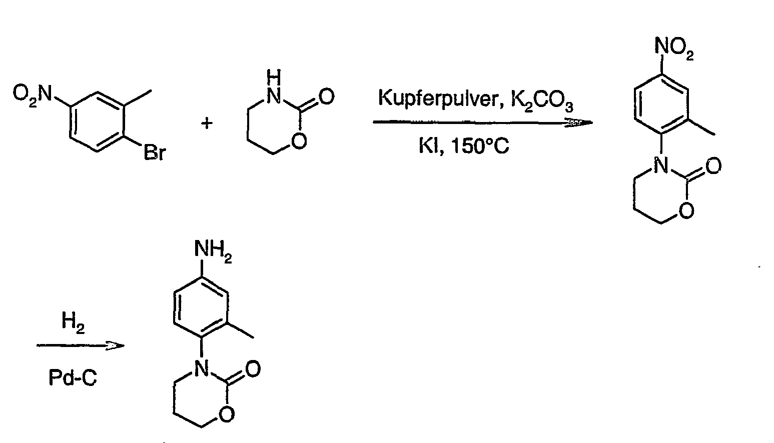

### 14.14 4-(4-Amino-phenyl)-morpholin-3-on

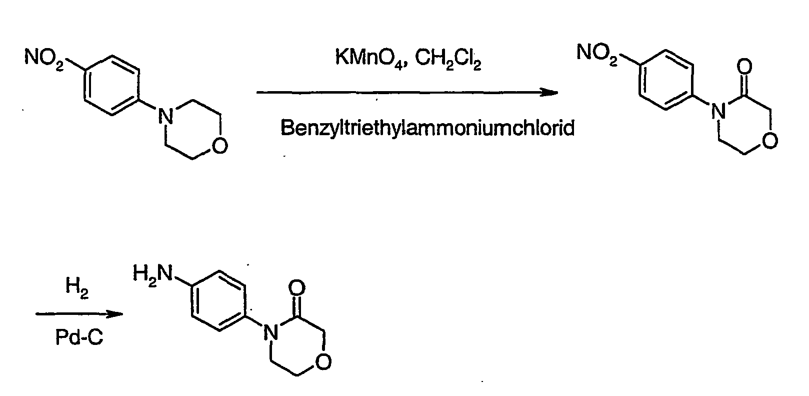

### 14.15 1-(4-Amino-phenyl)-pyridin-2-on

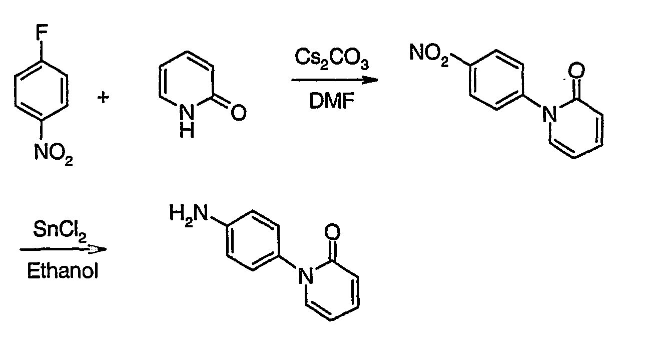

### 14.16 1-(4-Amino-2-methyl-phenyl)-piperidin-2-on

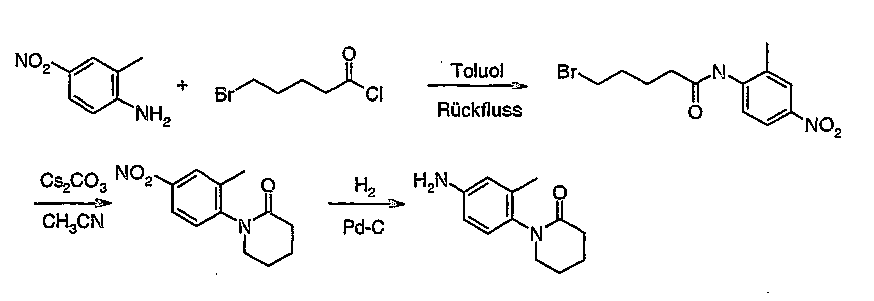

### 14.17 1-(4-Amino-phenyl)-1H-pyridin-4-on

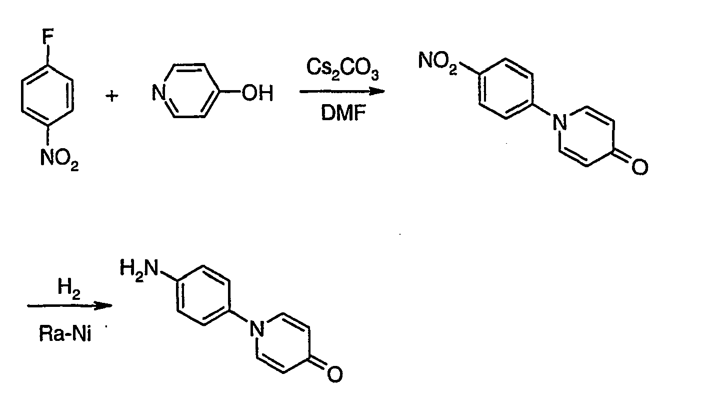

### 14.18 1-(4-Amino-phenyl)-4-tert.-butyloxycarbonyl-piperazin-2-on

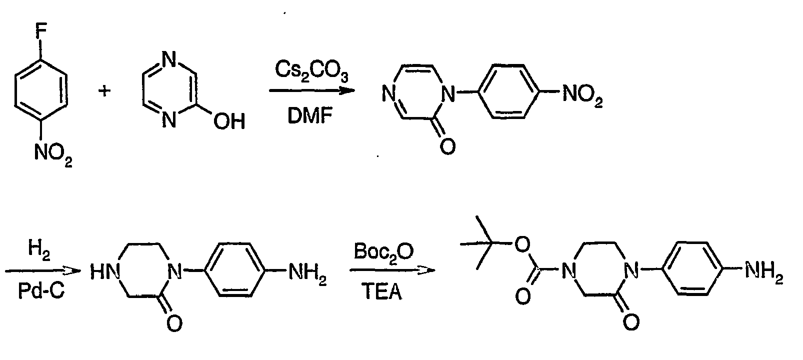

### 14.19 1-(3-Aminophenyl)-piperidin-2-on

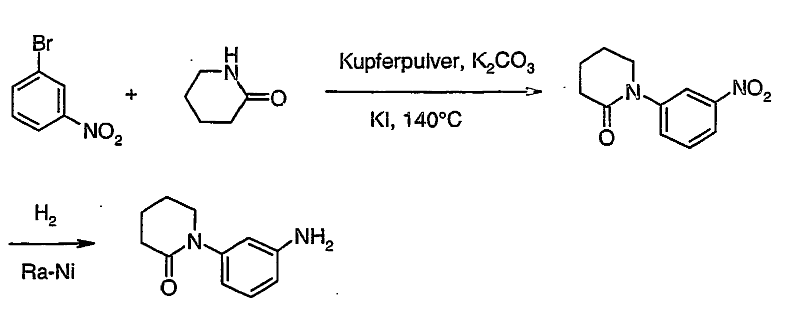

### 14.20 1-(4-Amino-phenyl)-2-caprolactam

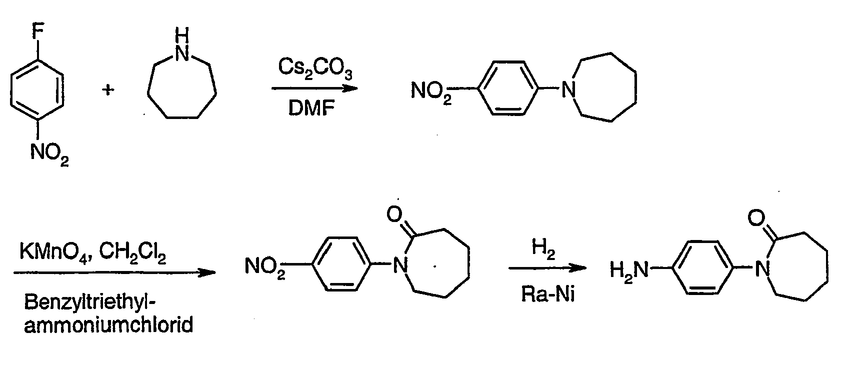

### 14.21 1-(4-Amino-3-fluor-phenyl)-piperidin-2-on

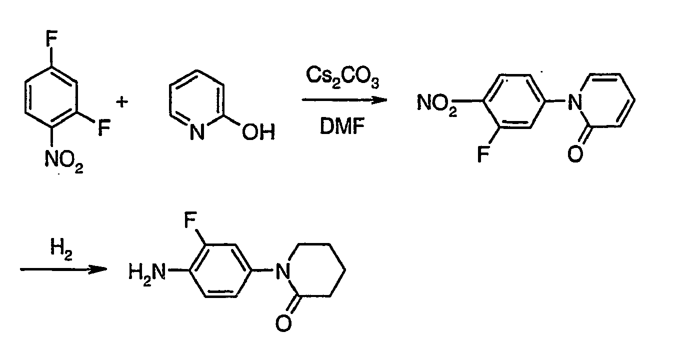

### 14.22 1-(4-Amino-2-fluor-phenyl)-piperidin-2-on

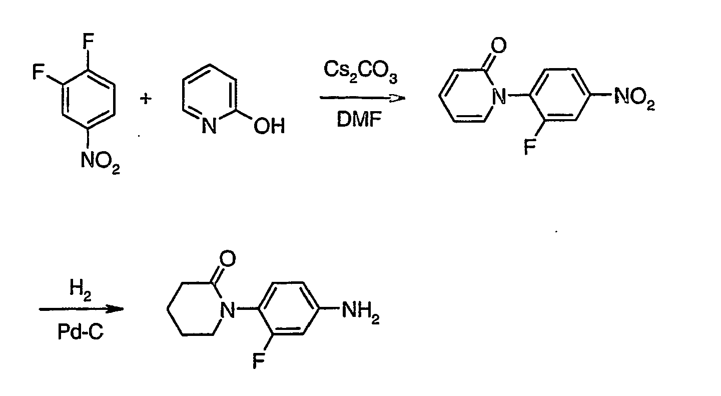

### 14.23 1-(4-Amino-2-fluor)-2-caprolactam

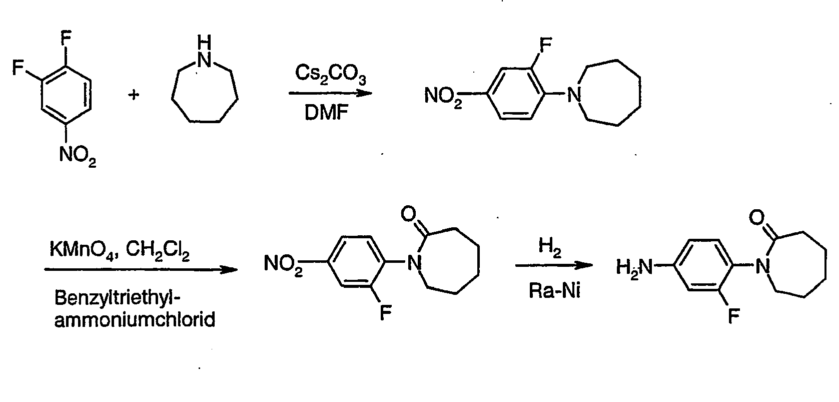

### Pharmakologische Daten

Affinität zu Rezeptoren

**Tabelle 1**

| Verbindung Nr. | FXa-IC₅₀ [nM] | TF/FVIIa-IC₅₀ [M] |
|---|---|---|
| "A1" | 75.0 | 160.0 |
| "A2" | 120.0 | 250.0 |
| "A3" | 55.0 | 120.0 |
| | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 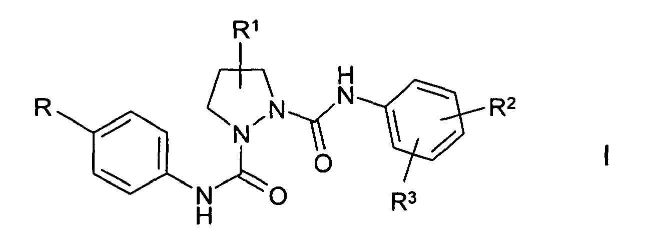 worin
R Hal oder -C≡C-H,
R¹ H, =O, OH, OA, A-COO-, Ph-(CH₂)ₙ-COO-, Cycloalkyl-(CH₂)ₙ-COO-,
Ph unsubstituiertes Phenyl,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
A unverzweigtes, verzweigtes oder cylisches Alkyl mit 1-10 C-Atomen, worin auch 1-7 H-Atome durch F und/oder Chlor ersetzt sein können,
Hal F, Cl, Br oder I,
n 0, 1, 2, 3 oder 4,
bedeuten,
sowie ihre pharmazeutisch verwendbaren Derivate, Salze, Solvate und Stereoisomere, einschließlich deren Mischungen in allen Verhältnissen.

2. Verbindungen nach Anspruch 1 ausgewählt aus der Gruppe
Pyrazolidin-1,2-dicarbonsäure-1-[(4-ethinyl-phenyl)-amid]-2-{[3-chlor-4-(3-oxo-morpholin-4-yl)-phenyl]-amid},
Pyrazolidin-1,2-dicarbonsäure-1-[(4-chlorphenyl)-amid]-2-{[3-chlor-4-(3-oxo-morpholin-4-yl)-phenyl]-amid},
4-Hydroxy-pyrazolidin-1,2-dicarbonsäure-1-[(4-chlorphenyl)-amid]-2-{[4-(3-oxo-morpholin-4-yl)-phenyl]-amid},
4-Hydroxy-pyrazolidin-1,2-dicarbonsäure-1-[(4-chlorphenyl)-amid]-2-{[4-(2-oxo-2*H*-pyridin-1-yl)-phenyl]-amid},
4-Hydroxy-pyrazolidin-1,2-dicarbonsäure-1 -[(4-chlorphenyl)-amid]-2-{[4-(2-oxo-piperidin-1-yl)-phenyl]-amid},
4-Hydroxy-pyrazolidin-1,2-dicarbonsäure-1-[(4-chlorphenyl)-amid]-2-{[3-methyl-4-(3-oxo-morpholin-4-yl)-phenyl]-amid},
4-Hydroxy-pyrazolidin-1,2-dicarbonsäure-1-[(4-chlorphenyl)-amid]-2-{[3-methyl-4-(2-oxo-pyrrolidin-yl)-phenyl]-amid},
4-Hydroxy-pyrazolidin-1,2-dicarbonsäure-1-[(4-chlorphenyl)-amid]-2-{[3-fluor-4-(3-oxo-morpholin-4-yl)-phenyl]-amid},
4-Hydroxy-pyrazolidin-1,2-dicarbonsäure-1-[(4-chlorphenyl)-amid]-2-{[3-chlor-4-(3-oxo-morpholin-4-yl)-phenyl]-amid},
4-Hydroxy-pyrazolidin-1,2-dicarbonsäure-1-[(4-chlorphenyl)-amid]-2-{[3-fluor-4-(2-oxo-pyrrolidin-yl)-phenyl]-amid},
4-Hydroxy-pyrazolidin-1,2-dicarbonsäure-1-[(4-chlorphenyl)-amid]-2-{[3-chlor-4-(2-oxo-2*H*-pyridin-1-yl)-phenyl]-amid},
4-Hydroxy-pyrazolidin-1,2-dicarbonsäure-1-[(4-chlorphenyl)-amid]-2-{[4-(2-aza-bicyclo[2.2.2]-octan-3-on-2-yl)-phenyl]-amid},
4-Hydroxy-pyrazolidin-1,2-dicarbonsäure-1-[(4-chlorphenyl)-amid]-2-{[3-trifluormethyl-4-(2-aza-bicyclo[2.2.2]-octan-3-on-2-yl)-phenyl]-amid},
4-Hydroxy-pyrazolidin-1,2-dicarbonsäure-1-[(4-chlorphenyl)-amid]-2-{[3-chlor-4-(2-aza-bicyclo[2.2.2]-octan-3-on-2-yl)-phenyl]-amid},
Pyrazolidin-1,2-dicarbonsäure-1-[(4-chlorphenyl)-amid]-2-{[4-(2-oxo-2*H*-pyridin-1-yl)-phenyl]-amid},
Pyrazolidin-1,2-dicarbonsäure-1-[(4-chlorphenyl)-amid]-2-{[3-methyl-4-(3-oxo-morpholin-4-yl)-phenyl]-amid},
Pyrazolidin-1,2-dicarbonsäure-1-[(4-chlorphenyl)-amid]-2-{[3-fluor-4-(3-oxo-morpholin-4-yl)-phenyl]-amid},
Pyrazolidin-1,2-dicarbonsäure-1-[(4-chlorphenyl)-amid]-2-{[3-chlor-4-(2-oxo-2*H*-pyridin-1-yl)-phenyl]-amid},
Pyrazolidin-1,2-dicarbonsäure-1-[(4-chlorphenyl)-amid]-2-{[3-chlor-4-(2-aza-bicyclo[2.2.2]-octan-3-on-2-yl)-phenyl]-amid},
Pyrazolidin-1,2-dicarbonsäure-1-[(4-chlorphenyl)-amid]-2-{[3-methyl-4-(2-oxo-pyrrolidin-yl)-phenyl]-amid},
4-Oxo-pyrazolidin-1,2-dicarbonsäure-1-[(4-chlorphenyl)-amid]-2-{[4-(3-oxo-morpholin-4-yl)-phenyl]-amid},
Pyrazolidin-1,2-dicarbonsäure-1-[(4-chlorphenyl)-amid]-2-{[4-(2-oxo-piperidin-yl)-phenyl]-amid},
Pyrazolidin-1,2-dicarbonsäure-1-[(4-chlorphenyl)-amid]-2-{[4-(3-oxo-morpholin-4-yl)-phenyl]-amid},
Pyrazolidin-1,2-dicarbonsäure-1-[(4-chlorphenyl)-amid]-2-{[2-fluor-4-(3-oxo-morpholin-4-yl)-phenyl]-amid},
Pyrazolidin-1,2-dicarbonsäure-1-[(4-chlorphenyl)-amid]-2-{[3-trifluormethyl-4-(2-aza-bicyclo[2.2.2]-octan-3-on-2-yl)-phenyl]-amid},
Pyrazolidin-1,2-dicarbonsäure-1-[(4-chlorphenyl)-amid]-2-{[4-(2-aza-bicyclo[2.2.2]-octan-3-on-2-yl)-phenyl]-amid},
Pyrazolidin-1,2-dicarbonsäure-1-[(4-chlorphenyl)-amid]-2-{[4-(2-oxo-[1,3]oxazinan-3-yl)-phenyl]-amid},
Pyrazolidin-1,2-dicarbonsäure-1-[(4-ethinylphenyl)-amid]-2-{[4-(2-oxo-2*H*-pyridin-1-yl)-phenyl]-amid},
Pyrazolidin-1,2-dicarbonsäure-1-[(4-ethinyl-phenyl)-amid]-2-{[3-methyl-4-(3-oxo-morpholin-4-yl)-phenyl]-amid},
4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
(R)-4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
(R)-4-Hydroxy-pyrazolid in-1, 2-dicarbonsäure-1-[(4-ethinyl-phenyl)-amid]-2-{[4-(2-oxo-2*H*-pyridin-1-yl)-phenyl]-amid},
(R)-4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
(S)-4-Hydroxy-pyrazolidin-1,2-dicarbonsäure-1-[(4-ethinyl-phenyl)-amid]-2-{[3-chlor-4-(3-oxo-morpholin-4-yl)-phenyl]-amid},
(S)-4-Hydroxy-pyrazolidin-1,2-dicarbonsäure-1-[(4-ethinyl-phenyl)-amid]-2-{[4-(2-oxo-2*H*-pyridin-1-yl)-phenyl]-amid},
(S)-4-Hydroxy-pyrazolidin-1,2-dicarbonsäure-1-[(4-ethinyl-phenyl)-amid]-2-{[3-methyl-4-(3-oxo-morpholin-4-yl)-phenyl]-amid},
4-Acetoxy-pyrazolidin-1,2-dicarbonsäure-1-[(4-ethinyl-phenyl)-amid]-2-{[3-chlor-4-(3-oxo-morpholin-4-yl)-phenyl]-amid},
4-Benzylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Benzoyloxy-pyrazolidin-1,2-dicarbonsäure-1-[(4-ethinyl-phenyl)-amid]-2-{[3-methyl-4-(3-oxo-morpholin-4-yl)-phenyl]-amid},
4-*tert*.-Butylcarbonyloxy-pyrazolidin-1,2-dicarbonsäure-1-[(4-ethinyl-phenyl)-amid]-2-{[3-chlor-4-(3-oxo-morpholin-4-yl)-phenyl]-amid},
4-Isobutylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Cyclohexylmethylcarbonyloxy-pyrazolidin-1,2-dicarbonsäure-1-[(4-ethinyl-phenyl)-amid]-2-{[3-methyl-4-(3-oxo-morpholin-4-yl)-phenyl]-amid},
4-Cyclopentylcarbonyloxy-pyrazolidin-1,2-dicarbonsäure-1-[(4-ethinyl-phenyl)-amid]-2-{[3-chlor-4-(3-oxo-morpholin-4-yl)-phenyl]-amid},
4-Cyclopropylmethylcarbonyloxy-pyrazolidin-1,2-dicarbonsäure-1-[(4-ethinyl-phenyl)-amid]-2-{[4-(2-oxo-2*H*-pyridin-1-yl)-phenyl]-amid},
4-Cyclobutylcarbonyloxy-pyrazolidin-1,2-dicarbonsäure-1-[(4-ethinyl-phenyl)-amid]-2-{[3-methyl-4-(3-oxo-morpholin-4-yl)-phenyl]-amid},
Pyrazolidin-1,2-dicarbonsäure-1-[(4-bromphenyl)-amid]-2-{[4-(2-oxo-2*H*-pyridin-1-yl)-phenyl]-amid},
4-Hydroxy-pyrazolidin-1,2-dicarbonsäure-1-[(4-bromphenyl)-amid]-2-{[4-(2-oxo-2*H*-pyridin-1-yl)-phenyl]-amid},
(S)-4-Hydroxy-pyrazolidin-1,2-dicarbonsäure-1-[(4-bromphenyl)-amid]-2-{[4-(2-oxo-2*H*-pyridin-1-yl)-phenyl]-amid},
(R)-4-Hydroxy-pyrazolidin-1,2-dicarbonsäure-1-[(4-bromphenyl)-amid]-2-{[4-(2-oxo-2*H*-pyridin-1-yl)-phenyl]-amid},
sowie ihre pharmazeutisch verwendbaren Derivate, Salze, Solvate und Stereoisomere, einschließlich deren Mischungen in allen Verhältnissen.

3. Verfahren zur Herstellung von Verbindungen der Formel I nach den Ansprüchen 1-2 sowie ihrer pharmazeutisch verwendbaren Derivate, Salze, Solvate und Stereoisomere, **dadurch gekennzeichnet, daß** man
a) eine Verbindung der Formel II 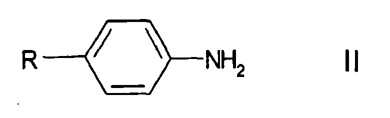 worin R die in Anspruch 1 angegebene Bedeutung hat,
mit einem Chloroformiatderivat zu einem intermediären Carbamatderivat umsetzt,
das anschließend mit einer Verbindung der Formel III-1 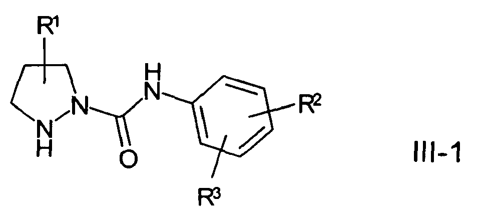 worin
R¹, R² und R³ die in Anspruch 1 angegebene Bedeutung haben,
und falls R¹ OH bedeutet, die OH-Gruppe gegebenenfalls geschützt vorliegt,
umgesetzt wird,
und anschließend gegebenenfalls die OH-Schutzgruppe abgespalten
wird,
oder
b) eine Verbindung der Formel IV 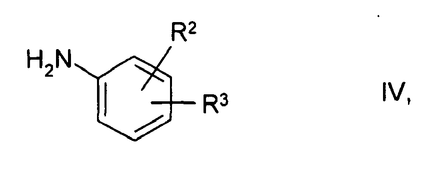
worin R² und R³ die in Anspruch 1 angegebene Bedeutung haben, mit einem Chloroformiatderivat zu einem intermediären Carbamatderivat umsetzt,
das anschließend mit einer Verbindung der Formel III-2 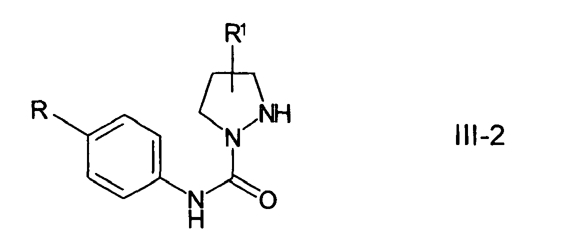 worin R und R¹ die in Anspruch 1 angegebene Bedeutung haben, und falls R¹ OH bedeutet, die OH-Gruppe gegebenenfalls geschützt vorliegt,
umgesetzt wird,
und anschließend gegebenenfalls die OH-Schutzgruppe abgespalten
wird,
und/oder
eine Base oder Säure der Formel I in eines ihrer Salze umwandelt.

4. Arzneimittel, enthaltend mindestens eine Verbindung der Formel I nach Anspruch 1 oder 2 und/oder ihre pharmazeutisch verwendbaren Derivate, Salze, Solvate und Stereoisomere, einschließlich deren Mischungen in allen Verhältnissen, sowie gegebenenfalls Träger- und/oder Hilfsstoffe.

5. Arzneimittel enthaltend mindestens eine Verbindung der Formel I gemäß Anspruch 1 oder 2 und/oder ihre pharmazeutisch verwendbaren Derivate, Salze, Solvate und Stereoisomere, einschließlich deren Mischungen in allen Verhältnissen, und mindestens einen weiteren Arzneimittelwirkstoff.

6. Verwendung von Verbindungen gemäß Anspruch 1 oder 2 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

7. Set (Kit), bestehend aus getrennten Packungen von
(a) einer wirksamen Menge an einer Verbindung der Formel I gemäß Anspruch 1 oder 2 und/oder ihrer pharmazeutisch verwendbaren Derivate, Salze, Solvate und Stereoisomere, einschließlich deren Mischungen in allen Verhältnissen,
und
(b) einer wirksamen Menge eines weiteren Arzneimittelswirkstoffs.

8. Verwendung von Verbindungen der Formel I gemäß Anspruch 1 oder 2 und/oder ihrer pharmazeutisch verwendbaren Derivate, Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

9. Zwischenverbindungen der Formel III-1 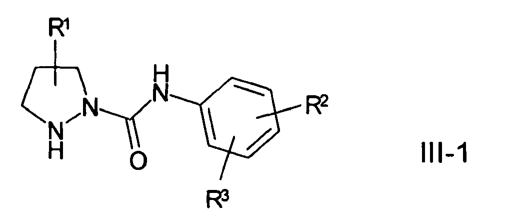 worin
R¹ H, =O, OR⁶, OA, A-COO-, Ph-(CH₂)ₙ-COO- oder Cycloalkyl-(CH₂)ₙ-COO-,
Ph unsubstituiertes Phenyl,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
R⁶ eine OH-Schutzgruppe,
A unverzweigtes, verzweigtes oder cylisches Alkyl mit 1-10 C-Atomen, worin auch 1-7 H-Atome durch F und/oder Chlor ersetzt sein können,
Hal F, Cl, Br oder I,
n 0, 1, 2, 3 oder 4,
bedeuten,
sowie deren Isomere und Salze.

10. Zwischenverbindungen nach Anspruch 9,
worin
R¹ H, =O oder OR⁶,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl, 4*H*-[1,4]Oxazin-4-yl,
R⁶ eine Alkylsilyl-Schutzgruppe,
A unverzweigtes, verzweigtes oder cylisches Alkyl mit 1-10 C-Atomen, worin auch 1-7 H-Atome durch F und/oder Chlor ersetzt sein können,
Hal F, Cl, Br oder I,
n 0, 1, 2, 3 oder 4,
bedeuten,
sowie deren Isomere und Salze.

11. Zwischenverbindungen der Formel III-2 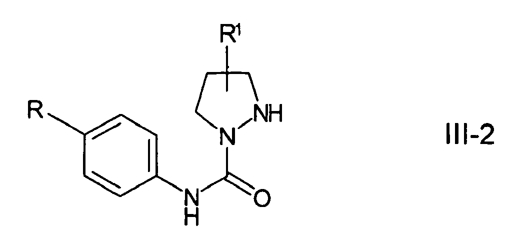 worin
R Hal oder -C≡C-H,
R¹ H, =O oder OR⁶,
R⁶ eine Alkylsilyl-Schutzgruppe,
Hal F, Cl, Br oder I,
bedeuten,
wobei, falls R¹ H bedeutet, R nicht Cl bedeutet,
sowie deren Isomere und Salze.

12. Zwischenverbindungen der Formel VI 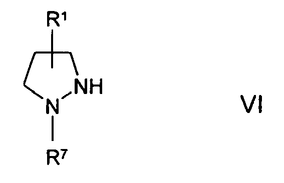 worin
R¹ OH oder OR⁶,
R⁶ eine Silyl-Schutzgruppe,
R⁷ *tert*.-Butyloxycarbonyl (BOC) oder Benzyloxycarbonyl (Z)
bedeuten,
sowie deren Isomere.

13. Verfahren zur Herstellung von Verbindungen der Formel VI 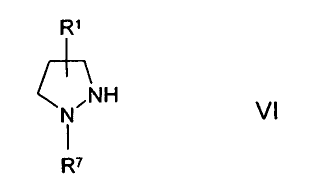 worin
R¹ OH oder OR⁶,
R⁶ eine Silylschutzgruppe,
R⁷ *tert*.-Butyloxycarbonyl (BOC) oder Benzyloxycarbonyl (Z)
bedeuten,
sowie deren Isomere,
erhältlich durch Umsetzung einer Verbindung der Formel VII
R⁷-NHNH₂ VII,
worin R⁷ BOC oder Z bedeutet,
mit silylgeschütztem 1,3-Dibrom-propan-2-ol,
und gegebenenfalls, anschließender Abspaltung der Schutzgruppe.

## Claims

1. Compounds of the formula I 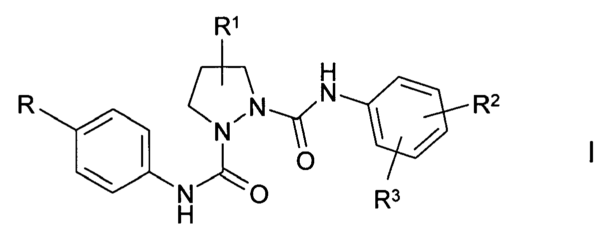 in which
R denotes Hal or -C≡C-H,
R¹ denotes H, =O, OH, OA, A-COO-, Ph-(CH₂)ₙ-COO-, cycloalkyl-(CH₂)ₙ-COO-,
Ph denotes unsubstituted phenyl,
R² denotes H, Hal or A,
R³ denotes 2-oxopiperidin-1-yl, 2-oxopyrrolidin-1-yl, 2-oxo-1*H*-pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2,6-dioxopiperidin-1-yl, 2-oxopiperazin-1-yl, 2,6-dioxo-piperazin-1-yl, 2,5-dioxopyrrolidin-1-yl, 2-oxo-1,3-oxa-zolidin-3-yl, 3-oxo-2*H*-pyridazin-2-yl, 2-caprolactam-1-yl (= 2-oxoazepan-1-yl), 2-azabicyclo[2.2.2]-octan-3-on-2-yl, 5,6-dihydro-1*H*-pyrimidin-2-oxo-1-yl, 2-oxo-1,3-oxazinan-3-yl or 4*H*-1,4-oxazin-4-yl,
A denotes unbranched, branched or cyclic alkyl having 1-10 C atoms, in which 1-7 H atoms may also be replaced by F and/or chlorine,
Hal denotes F, Cl, Br or I,
n denotes 0, 1, 2, 3 or 4,
and pharmaceutically usable derivatives, salts, solvates and stereoisomers thereof, including mixtures thereof in all ratios.

2. Compounds according to Claim 1 selected from the group
1-N-[(4-ethynylphenyl)]-2-N-{[3-chloro-4-(3-oxomorpholin-4-yl)phenyl]}pyrazolidine-1,2-dicarboxamide,
1-N-[(4-chlorophenyl)]-2-N-{[3-chloro-4-(3-oxomorpholin-4-yl)-phenyl]}pyrazolidine-1,2-dicarboxamide,
1-N-[(4-chlorophenyl)]-2-N-{[4-(3-oxomorpholin-4-yl)phenyl]}-4-hydroxypyrazolidine-1,2-dicarboxamide,
1-N-[(4-chlorophenyl)]-2-N-{[4-(2-oxo-2*H*-pyridin-1-yl)phenyl]}-4-hydroxypyrazolidine-1,2-dicarboxamide,
1-N-[(4-chlorophenyl)]-2-N-{[4-(2-oxopiperidin-1-yl)phenyl]}-4-hydroxypyrazolidine-1,2-dicarboxamide,
1-N-[(4-chlorophenyl)]-2-N-{[3-methyl-4-(3-oxomorpholin-4-yl)-phenyl]}-4-hydroxypyrazolidine-1,2-dicarboxamide,
1-N-[(4-chlorophenyl)]-2-N-{[3-methyl-4-(2-oxopyrrolidinyl)-phenyl]}-4-hydroxypyrazolidine-1,2-dicarboxamide,
1-N-[(4-chlorophenyl)]-2-N-{[3-fluoro-4-(3-oxomorpholin-4-yl)-phenyl]}-4-hydroxypyrazolidine-1,2-dicarboxamide,
1-N-[(4-chlorophenyl)]-2-N-{[3-chloro-4-(3-oxomorpholin-4-yl)-phenyl]}-4-hydroxypyrazolidine-1,2-dicarboxamide,
1-N-[(4-chlorophenyl)]-2-N-{[3-fluoro-4-(2-oxopyrrolidinyl)-phenyl]}-4-hydroxypyrazolidine-1,2-dicarboxamide,
1-N-[(4-chlorophenyl)]-2-N-{[3-chloro-4-(2-oxo-2*H*-pyridin-1-yl)-phenyl]}-4-hydroxypyrazolidine-1,2-dicarboxamide,
1-N-[(4-chlorophenyl)]-2-N-{[4-(2-azabicyclo[2.2.2]-octan-3-on-2-yl)phenyl]}-4-hydroxypyrazolidine-1,2-dicarboxamide,
1-N-[(4-chlorophenyl)]-2-N-{[3-trifluoromethyl-4-(2-azabicyclo-[2.2.2]-octan-3-on-2-yl)phenyl]}-4-hydroxypyrazolidine-1,2-dicarboxamide,
1-N-[(4-chlorophenyl)]-2-N-{[3-chloro-4-(2-azabicyclo[2.2.2]-octan-3-on-2-yl)phenyl]}-4-hydroxypyrazolidine-1,2-dicarboxamide,
1-N-[(4-chlorophenyl)]-2-N-{[4-(2-oxo-2*H*-pyridin-1-yl)phenyl]}-pyrazolidine-1,2-dicarboxamide,
1-N-[(4-chlorophenyl)]-2-N-{[3-methyl-4-(3-oxomorpholin-4-yl)-phenyl]}pyrazolidine-1,2-dicarboxamide,
1-N-[(4-chlorophenyl)]-2-N-{[3-fluoro-4-(3-oxomorpholin-4-yl)-phenyl]}pyrazolidine-1,2-dicarboxamide,
1-N-[(4-chlorophenyl)]-2-N-{[3-chloro-4-(2-oxo-2*H*-pyridin-1-yl)-phenyl]}pyrazolidine-1,2-dicarboxamide,
1-N-[(4-chlorophenyl)]-2-N-{[3-chloro-4-(2-azabicyclo[2.2.2]-octan-3-on-2-yl)phenyl]}pyrazolidine-1,2-dicarboxamide,
1-N-[(4-chlorophenyl)]-2-N-{[3-methyl-4-(2-oxopyrrolidinyl)-phenyl]}pyrazolidine-1,2-dicarboxamide,
1-N-[(4-chlorophenyl)]-2-N-{[4-(3-oxomorpholin-4-yl)phenyl]}-4-oxopyrazolidine-1,2-dicarboxamide,
1-N-[(4-chlorophenyl)]-2-N-{[4-(2-oxopiperidinyl)phenyl]}pyrazolidine-1,2-dicarboxamide,
1-N-[(4-chlorophenyl)]-2-N-{[4-(3-oxomorpholin-4-yl)phenyl]}-pyrazolidine-1,2-dicarboxamide,
1-N-[(4-chlorophenyl)]-2-N-{[2-fluoro-4-(3-oxomorpholin-4-yl)-phenyl]}pyrazolidine-1,2-dicarboxamide,
1-N-[(4-chlorophenyl)]-2-N-{[3-trifluoromethyl-4-(2-azabicyclo-[2.2.2]-octan-3-on-2-yl)phenyl]}pyrazolidine-1,2-dicarboxamide,
1-N-[(4-chlorophenyl)]-2-N-{[4-(2-azabicyclo[2.2.2]-octan-3-on-2-yl)phenyl]}pyrazolidine-1,2-dicarboxamide,
1-N-[(4-chlorophenyl)]-2-N-{[4-(2-oxo-1,3-oxazinan-3-yl)phenyl]}-pyrazolidine-1,2-dicarboxamide,
1-N-[(4-ethynylphenyl)]-2-N-{[4-(2-oxo-2*H*-pyridin-1-yl)phenyl]}-pyrazolidine-1,2-dicarboxamide,
1-N-[(4-ethynylphenyl)]-2-N-{[3-methyl-4-(3-oxomorpholin-4-yl)-phenyl]}pyrazolidine-1,2-dicarboxamide,
1-N-[(4-ethynylphenyl)]-2-N-{[3-chloro-4-(3-oxomorpholin-4-yl)-phenyl]}-4-hydroxypyrazolidine-1,2-dicarboxamide,
1-N-[(4-ethynylphenyl)]-2-N-{[4-(2-oxo-2*H*-pyridin-1-yl)phenyl]}-4-hydroxypyrazolidine-1,2-dicarboxamide,
1-N-[(4-ethynylphenyl)]-2-N-{[3-methyl-4-(3-oxomorpholin-4-yl)-phenyl]}-4-hydroxypyrazolidine-1,2-dicarboxamide,
1-N-[(4-ethynylphenyl)]-2-N-{[3-chloro-4-(3-oxomorpholin-4-yl)-phenyl]}-(R)-4-hydroxypyrazolidine-1,2-dicarboxamide,
1-N-[(4-ethynylphenyl)]-2-N-{[4-(2-oxo-2*H*-pyridin-1-yl)phenyl]}-(R)-4-hydroxypyrazolidine-1,2-dicarboxamide,
1-N-[(4-ethynylphenyl)]-2-N-{[3-methyl-4-(3-oxomorpholin-4-yl)-phenyl]}-(R)-4-hydroxypyrazolidine-1,2-dicarboxamide,
1-N-[(4-ethynylphenyl)]-2-N-{[3-chloro-4-(3-oxomorpholin-4-yl)-phenyl]}-(S)-4-hydroxypyrazolidine-1,2-dicarboxamide,
1-N-[(4-ethynylphenyl)]-2-N-{[4-(2-oxo-2*H*-pyridin-1-yl)phenyl]}-(S)-4-hydroxypyrazolidine-1,2-dicarboxamide,
1-N-[(4-ethynylphenyl)]-2-N-{[3-methyl-4-(3-oxomorpholin-4-yl)-phenyl]}-(S)-4-hydroxypyrazolidine-1,2-dicarboxamide,
1-N-[(4-ethynylphenyl)]-2-N-{[3-chloro-4-(3-oxomorpholin-4-yl)-phenyl]}-4-acetoxypyrazolidine-1,2-dicarboxamide,
1-N-[(4-ethynylphenyl)]-2-N-{[4-(2-oxo-2*H*-pyridin-1-yl)phenyl]}-4-benzylcarbonyloxypyrazolidine-1,2-dicarboxamide,
1-N-[(4-ethynylphenyl)]-2-N-{[3-methyl-4-(3-oxomorpholin-4-yl)-phenyl]}-4-benzoyloxypyrazolidine-1,2-dicarboxamide,
1-N-[(4-ethynylphenyl)]-2-N-{[3-chloro-4-(3-oxomorpholin-4-yl)-phenyl]}-4-*tert*-butylcarbonyloxypyrazolidine-1,2-dicarboxamide,
1-N-[(4-ethynylphenyl)]-2-N-{[4-(2-oxo-2*H*-pyridin-1-yl)phenyl]}-4-isobutylcarbonyloxypyrazolidine-1,2-dicarboxamide,
1-N-[(4-ethynylphenyl)]-2-N-{[3-methyl-4-(3-oxomorpholin-4-yl)-phenyl]}-4-cyclohexylmethylcarbonyloxypyrazolidine-1,2-dicarbox-amide,
1-N-[(4-ethynylphenyl)]-2-N-{[3-chloro-4-(3-oxomorpholin-4-yl)-phenyl]}-4-cyclopentylcarbonyloxypyrazolidine-1,2-dicarboxamide,
1-N-[(4-ethynylphenyl)]-2-N-{[4-(2-oxo-2*H*-pyridin-1-yl)phenyl]}-4-cyclopropylmethylcarbonyloxypyrazolidine-1,2-dicarboxamide,
1-N-[(4-ethynylphenyl)]-2-N-{[3-methyl-4-(3-oxomorpholin-4-yl)-phenyl]}-4-cyclobutylcarbonyloxypyrazolidine-1,2-dicarboxamide,
1-N-[(4-bromophenyl)]-2-N-{[4-(2-oxo-2*H*-pyridin-1-yl)phenyl]}-pyrazolidine-1,2-dicarboxamide,
1-N-[(4-bromophenyl)]-2-N-{[4-(2-oxo-2*H*-pyridin-1-yl)phenyl]}-4-hydroxypyrazolidine-1,2-dicarboxamide,
1-N-[(4-bromophenyl)]-2-N-{[4-(2-oxo-2*H*-pyridin-1-yl)phenyl]}-(S)-4-hydroxypyrazolidine-1,2-dicarboxamide,
1-N-[(4-bromophenyl)]-2-N-{[4-(2-oxo-2*H*-pyridin-1-yl)phenyl]}-(R)-4-hydroxypyrazolidine-1,2-dicarboxamide,
and pharmaceutically usable derivatives, salts, solvates and stereoisomers thereof, including mixtures thereof in all ratios.

3. Process for the preparation of compounds of the formula I according to Claims 1-2 and pharmaceutically usable derivatives, salts, solvates and stereoisomers thereof, **characterised in that**
a) a compound of the formula II 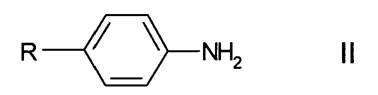 in which R has the meaning indicated in Claim 1,
is reacted with a chloroformate derivative to give an intermediate carbamate derivative,
which is subsequently reacted with a compound of the formula III-1 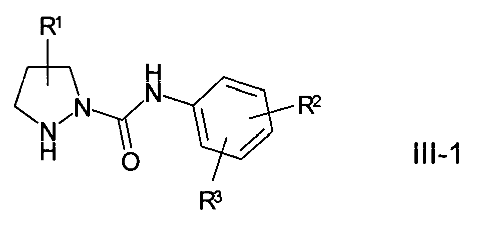 in which
R¹, R² and R³ have the meaning indicated in Claim 1,
and, if R¹ denotes OH, the OH group is optionally in protected form,
and subsequently, if desired, the OH-protecting group is removed,
or
b) a compound of the formula IV 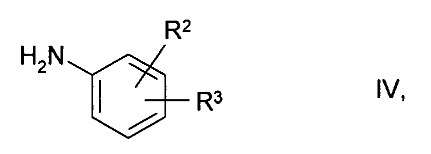
in which R² and R³ have the meaning indicated in Claim 1,
is reacted with a chloroformate derivative to give an intermediate carbamate derivative,
which is subsequently reacted with a compound of the formula III-2 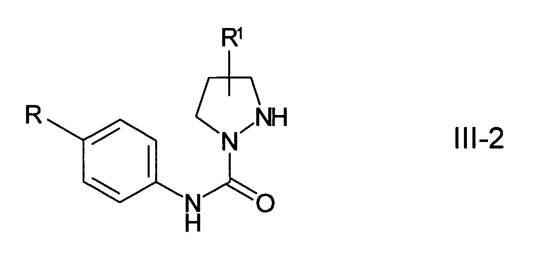 in which R and R¹ have the meaning indicated in Claim 1,
and, if R¹ denotes OH, the OH group is optionally in protected form,
and subsequently, if desired, the OH-protecting group is removed,
and/or
a base or acid of the formula I is converted into one of its salts.

4. Medicaments comprising at least one compound of the formula I according to Claim 1 or 2 and/or pharmaceutically usable derivatives, salts, solvates and stereoisomers thereof, including mixtures thereof in all ratios, and optionally excipients and/or adjuvants.

5. Medicaments comprising at least one compound of the formula I according to Claims 1 or 2 and/or pharmaceutically usable derivatives, salts, solvates and stereoisomers thereof, including mixtures thereof in all ratios, and at least one further medicament active ingredient.

6. Use of compounds according to Claim 1 or 2 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

7. Set (kit) consisting of separate packs of
(a) an effective amount of a compound of the formula I according to Claim 1 or 2 and/or pharmaceutically usable derivatives, salts, solvates and stereoisomers thereof, including mixtures thereof in all ratios,
and
(b) an effective amount of a further medicament active ingredient.

8. Use of compounds of the formula I according to Claim 1 or 2 and/or pharmaceutically usable derivatives, salts, solvates and stereoisomers thereof, including mixtures thereof in all ratios,
for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases,
in combination with at least one further medicament active ingredient.

9. Intermediate compounds of the formula III-1 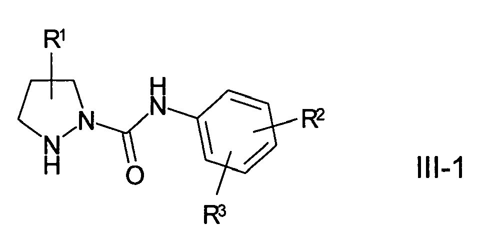 in which
R¹ denotes H, =O, OR⁶, OA, A-COO-, Ph-(CH₂)ₙ-COO- or cycloalkyl-(CH₂)ₙ-COO-,
Ph denotes unsubstituted phenyl,
R² denotes H, Hal or A,
R³ denotes 2-oxopiperidin-1-yl, 2-oxopyrrolidin-1-yl, 2-oxo-1*H*-pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2,6-dioxopiperidin-1-yl, 2-oxopiperazin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-caprolactam-1-yl (= 2-oxo-azepan-1-yl), 2-azabicyclo[2.2.2]-octan-3-on-2-yl, 5,6-dihydro-1*H*-pyrimidin-2-oxo-1-yl, 2-oxo-1,3-oxazinan-3-yl or 4*H*-1,4-oxazin-4-yl,
R⁶ denotes an OH-protecting group,
A denotes unbranched, branched or cyclic alkyl having 1-10 C atoms, in which 1-7 H atoms may also be replaced by F and/or chlorine,
Hal denotes F, Cl, Br or I,
n denotes 0, 1, 2, 3 or 4,
and isomers and salts thereof.

10. Intermediate compounds according to Claim 9,
in which
R¹ denotes H, =O or OR⁶,
R² denotes H, Hal or A,
R³ denotes 2-oxopiperidin-1-yl, 2-oxopyrrolidin-1-yl, 2-oxo-1*H*-pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2,6-dioxopiperidin-1-yl, 2-oxopiperazin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-caprolactam-1-yl (= 2-oxo-azepan-1-yl), 2-azabicyclo[2.2.2]-octan-3-on-2-yl, 5,6-dihydro-1*H*-pyrimidin-2-oxo-1-yl, 2-oxo-1,3-oxazinan-3-yl, 4*H*-1,4-oxazin-4-yl,
R⁶ denotes an alkylsilyl protecting group,
A denotes unbranched, branched or cyclic alkyl having 1-10 C atoms, in which 1-7 H atoms may also be replaced by F and/or chlorine,
Hal denotes F, Cl, Br or I,
n denotes 0, 1, 2, 3 or 4,
and isomers and salts thereof.

11. Intermediate compounds of the formula III-2 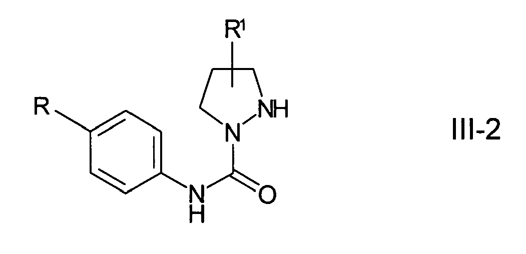 in which
R denotes Hal or -C≡C-H,
R¹ denotes H, =O or OR⁶,
R⁶ denotes an alkylsilyl protecting group,
Hal denotes F, Cl, Br or I,
where, if R¹ denotes H, R does not denote Cl,
and isomers and salts thereof.

12. Intermediate compounds of the formula VI 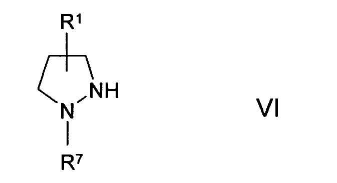 in which
R¹ denotes OH or OR⁶,
R⁶ denotes a silyl protecting group,
R⁷ denotes *tert*-butyloxycarbonyl (BOC) or benzyloxy carbonyl (Z),
and isomers thereof.

13. Process for the preparation of compounds of the formula VI 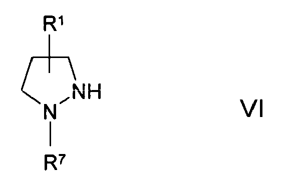 in which
R¹ denotes OH or OR⁶,
R⁶ denotes a silyl protecting group,
R⁷ denotes *tert*-butyloxycarbonyl (BOC) or benzyloxycarbonyl (Z),
and isomers thereof,
obtainable by reaction of a compound of the formula VII
R⁷-NHNH₂ VII,
in which R⁷ denotes BOC or Z,
with silyl-protected 1,3-dibromopropan-2-ol,
and optionally subsequent removal of the protecting group.

## Revendications

1. Composés de formule I 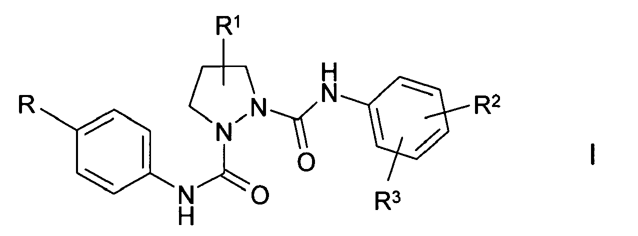 dans laquelle
R désigne Hal ou -C≡C-H,
R¹ désigne H, =O, OH, OA, A-COO-, Ph-(CH₂)ₙ-COO-, cycloalkyl-(CH₂)ₙ-COO-,
Ph désigne phényle non substitué,
R² désigne H, Hal ou A,
R³ désigne 2-oxopipéridin-1-yle, 2-oxopyrrolidin-1-yle, 2-oxo-1*H*-pyridin-1-yle, 3-oxomorpholin-4-yle, 4-oxo-1*H-*pyridin-1-yle, 2-oxo-1*H*-pyrazin-1-yle, 2-oxoimidazolidin-1-yle, 2,6-dioxopipéridin-1-yle, 2-oxopipérazin-1-yle, 2,6-dioxopipérazin-1-yle, 2,5-dioxopyrrolidin-1-yle, 2-oxo-1,3-oxazolidin-3-yle, 3-oxo-2*H*-pyridazin-2-yle, 2-caprolactam-1-yle (= 2-oxoazépan-1-yle), 2-aza-bicyclo[2.2.2]-octan-3-on-2-yle, 5,6-dihydro-1*H*-pyrimidin-2-oxo-1-yle, 2-oxo-1,3-oxazinan-3-yle ou 4*H*-1,4-oxazin-4-yle,
A désigne alkyle non ramifié, ramifié ou cyclique ayant 1-10 atomes de C, dans lequel 1-7 atomes de H peuvent également être remplacés par F et/ou chlore,
Hal désigne F, Cl, Br ou I,
n vaut 0, 1, 2, 3 ou 4,
et les dérivés, les sels, les solvats et les stéréoisomères pharmaceutiquement utilisables de ceux-ci, y compris des mélanges de ceux-ci selon des rapports quelconques.

2. Composés selon la revendication 1, choisis parmi le groupe
le 1-N-[(4-éthynylphényl)]-2-N-{[3-chloro-4-(3-oxomorpholin-4-yl)phényl]}pyrazolidine-1,2-dicarboxamide,
le 1-N-[(4-chlorophényl)]-2-N-{[3-chloro-4-(3-oxomorpholin-4-yl)-phényl]}pyrazolidine-1,2-dicarboxamide,
le 1-N-[(4-chlorophényl)]-2-N-{[4-(3-oxomorpholin-4-yl)phényl]}-4-hydroxypyrazolidine-1,2-dicarboxamide,
le 1-N-[(4-chlorophényl)]-2-N-{[4-(2-oxo-2*H*-pyridin-1-yl)phényl]}-4-hydroxypyrazolidine-1,2-dicarboxamide,
le 1-N-[(4-chlorophényl)]-2-N-{[4-(2-oxopipéridin-1-yl)phényl]}-4-hydroxypyrazolidine-1,2-dicarboxamide,
le 1-N-[(4-chlorophényl)]-2-N-{[3-méthyl-4-(3-oxomorpholin-4-yl)-phényl]}-4-hydroxypyrazolidine-1,2-dicarboxamide,
le 1-N-[(4-chlorophényl)]-2-N-{[3-méthyl-4-(2-oxopyrrolidinyl)-phényl]}-4-hydroxypyrazolidine-1,2-dicarboxamide,
le 1-N-[(4-chlorophényl)]-2-N-{[3-fluoro-4-(3-oxomorpholin-4-yl)-phényl]}-4-hydroxypyrazolidine-1,2-dicarboxamide,
le 1-N-[(4-chlorophényl)]-2-N-{[3-chloro-4-(3-oxomorpholin-4-yl)-phényl]}-4-hydroxypyrazolidine-1,2-dicarboxamide,
le 1-N-[(4-chlorophényl)]-2-N-{[3-fluoro-4-(2-oxopyrrolidinyl)-phényl]}-4-hydroxypyrazolidine-1,2-dicarboxamide,
le 1-N-[(4-chlorophényl)]-2-N-{[3-chloro-4-(2-oxo-2*H*-pyridin-1-yl)phényl]}-4-hydroxypyrazolidine-1,2-dicarboxamide,
le 1-N-[(4-chlorophényl)]-2-N-{[4-(2-azabicyclo[2.2.2]-octan-3-on-2-yl)phényl]}-4-hydroxypyrazolidine-1,2-dicarboxamide,
le 1-N-[(4-chlorophényl)]-2-N-{[3-trifluorométhyl-4-(2-azabicyclo-[2.2.2]-octan-3-on-2-yl)phényl]}-4-hydroxypyrazolidine-1,2-dicarboxamide,
le 1-N-[(4-chlorophényl)]-2-N-{[3-chloro-4-(2-azabicyclo[2.2.2]-octan-3-on-2-yl)phényl]}-4-hydroxypyrazolidine-1,2-dicarboxamide,
le 1-N-[(4-chlorophényl)]-2-N-{[4-(2-oxo-2*H*-pyridin-1-yl)phényl]}-pyrazolidine-1,2-dicarboxamide,
le 1-N-[(4-chlorophényl)]-2-N-{[3-méthyl-4-(3-oxomorpholin-4-yl)-phényl]}pyrazolidine-1,2-dicarboxamide,
le 1-N-[(4-chlorophényl)]-2-N-{[3-fluoro-4-(3-oxomorpholin-4-yl)-phényl]}pyrazolidine-1,2-dicarboxamide,
le 1-N-[(4-chlorophényl)]-2-N-{[3-chloro-4-(2-oxo-2*H*-pyridin-1-yl)phényl]}pyrazolidine-1,2-dicarboxamide,
le 1-N-[(4-chlorophényl)]-2-N-{[3-chloro-4-(2-azabicyclo[2.2.2]-octan-3-on-2-yl)phényl]}pyrazolidine-1,2-dicarboxamide,
le 1-N-[(4-chlorophényl)]-2-N-{[3-méthyl-4-(2-oxopyrrolidinyl)-phényl]}pyrazolidine-1,2-dicarboxamide,
le 1-N-[(4-chlorophényl)]-2-N-{[4-(3-oxomorpholin-4-yl)phényl]}-4-oxopyrazolidine-1,2-dicarboxamide,
le 1-N-[(4-chlorophényl)]-2-N-{[4-(2-oxopipéridinyl)phényl]}pyrazolidine-1,2-dicarboxamide,
le 1-N-[(4-chlorophényl)]-2-N-{[4-(3-oxomorpholin-4-yl)phényl]}-pyrazolidine-1,2-dicarboxamide,
le 1-N-[(4-chlorophényl)]-2-N-{[2-fluoro-4-(3-oxomorpholin-4-yl)-phényl]}pyrazolidine-1,2-dicarboxamide,
le 1-N-[(4-chlorophényl)]-2-N-{[3-trifluorométhyl-4-(2-azabicyclo-[2.2.2]-octan-3-on-2-yl)phényl]}pyrazolidine-1,2-dicarboxamide,
le 1-N-[(4-chlorophényl)]-2-N-{[4-(2-azabicyclo[2.2.2]-octan-3-on-2-yl)phényl]}pyrazolidine-1,2-dicarboxamide,
le 1-N-[(4-chlorophényl)]-2-N-{[4-(2-oxo-1,3-oxazinan-3-yl)-phényl]}pyrazolidine-1,2-dicarboxamide,
le 1-N-[(4-éthynylphényl)]-2-N-{[4-(2-oxo-2*H*-pyridin-1-yl)-phényl]}pyrazolidine-1,2-dicarboxamide,
le 1-N-[(4-éthynylphényl)]-2-N-{[3-méthyl-4-(3-oxomorpholin-4-yl)phényl]}pyrazolidine-1,2-dicarboxamide,
le 1-N-[(4-éthynylphényl)]-2-N-{[3-chloro-4-(3-oxomorpholin-4-yl)phényl]}-4-hydroxypyrazolidine-1,2-dicarboxamide,
le 1-N-[(4-éthynylphényl)]-2-N-{(4-(2-oxo-2*H*-pyridin-1-yl)-phényl]}-4-hydroxypyrazolidine-1,2-dicarboxamide,
le 1-N-[(4-éthynylphényl)]-2-N-{[3-méthyl-4-(3-oxomorpholin-4-yl)phényl]}-4-hydroxypyrazolidine-1,2-dicarboxamide,
le 1-N-[(4-éthynylphényl)]-2-N-{[3-chloro-4-(3-oxomorpholin-4-yl)phényl]}-(R)-4-hydroxypyrazolidine-1,2-dicarboxamide,
le 1-N-[(4-éthynylphényl)]-2-N-{[4-(2-oxo-2*H*-pyridin-1-yl)-phényl]}-(R)-4-hydroxypyrazolidine-1,2-dicarboxamide,
le 1-N-[(4-éthynylphényl)]-2-N-{[3-méthyl-4-(3-oxomorpholin-4-yl)phényl]}-(R)-4-hydroxypyrazolidine-1,2-dicarboxamide,
le 1-N-[(4-éthynylphényl)]-2-N-{[3-chloro-4-(3-oxomorpholin-4-yl)phényl]}-(S)-4-hydroxypyrazolidine-1,2-dicarboxamide,
le 1-N-[(4-éthynylphényl)]-2-N-{[4-(2-oxo-2*H*-pyridin-1-yl)-phényl]}-(S)-4-hydroxypyrazolidine-1,2-dicarboxamide,
le 1-N-[(4-éthynylphényl)]-2-N-{[3-méthyl-4-(3-oxomorpholin-4-yl)phényl]}-(S)-4-hydroxypyrazolidine-1,2-dicarboxamide,
le 1-N-[(4-éthynylphényl)]-2-N-{[3-chloro-4-(3-oxomorpholin-4-yl)phényl]}-4-acétoxypyrazolidine-1,2-dicarboxamide,
le 1-N-[(4-éthynylphényl)]-2-N-{[4-(2-oxo-2*H*-pyridin-1-yl)-phényl]}-4-benzylcarbonyloxypyrazolidine-1,2-dicarboxamide,
le 1-N-[(4-éthynylphényl)]-2-N-{[3-méthyl-4-(3-oxomorpholin-4-yl)phényl]}-4-benzoyloxypyrazolidine-1,2-dicarboxamide,
le 1-N-[(4-éthynylphényl)]-2-N-{[3-chloro-4-(3-oxomorpholin-4-yl)phényl]}-4-*tertio*-butylcarbonyloxypyrazolidine-1,2-dicarboxamide,
le 1-N-[(4-éthynylphényl)]-2-N-{[4-(2-oxo-2*H*-pyridin-1-yl)-phényl]}-4-isobutylcarbonyloxypyrazolidine-1,2-dicarboxamide,
le 1-N-[(4-éthynylphényl)]-2-N-{[3-méthyl-4-(3-oxomorpholin-4-yl)phényl]}-4-cyclohexylméthylcarbonyloxypyrazolidine-1,2-dicarboxamide,
le 1-N-[(4-éthynylphényl)]-2-N-{[3-chloro-4-(3-oxomorpholin-4-yl)phényl]}-4-cyclopentylcarbonyloxypyrazolidine-1,2-dicarboxamide,
le 1-N-[(4-éthynylphényl)]-2-N-{[4-(2-oxo-2*H*-pyridin-1-yl)-phényl]}-4-cyclopropylméthylcarbonyloxypyrazolidine-1,2-dicarboxamide,
le 1-N-[(4-éthynylphényl)]-2-N-{[3-méthyl-4-(3-oxomorpholin-4-yl)phényl]}-4-cyclobutylcarbonyloxypyrazolidine-1,2-dicarboxamide,
le 1-N-[(4-bromophényl)]-2-N-{[4-(2-oxo-2*H*-pyridin-1-yl)phényl]}-pyrazolidine-1,2-dicarboxamide,
le 1-N-[(4-bromophényl)]-2-N-{[4-(2-oxo-2*H*-pyridin-1-yl)phényl]}-4-hydroxypyrazolidine-1,2-dicarboxamide,
le 1-N-[(4-bromophényl)]-2-N-{[4-(2-oxo-2*H*-pyridin-1-yl)phényl]}-(S)-4-hydroxypyrazolidine-1,2-dicarboxamide,
le 1-N-[(4-bromophényl)]-2-N-{[4-(2-oxo-2*H*-pyridin-1-yl)phényl]}-(R)-4-hydroxypyrazolidine-1,2-dicarboxamide,
et les dérivés, les sels, les solvats et les stéréoisomères pharmaceutiquement utilisables de ceux-ci, y compris des mélanges de ceux-ci selon des rapports quelconques.

3. Procédé de préparation de composés de formule 1 selon les revendications 1-2 et de dérivés, de sels, de solvats et de stéréoisomères pharmaceutiquement utilisables de ceux-ci, **caractérisé en ce que**
a) un composé de formule II 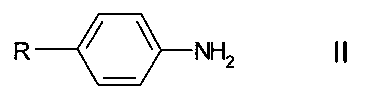 dans laquelle R a la signification indiquée selon la revendication 1, est réagi avec un dérivé de chloroformiate pour donner un dérivé de carbamate intermédiaire,
qui est ensuite réagi avec un composé de formule III-1 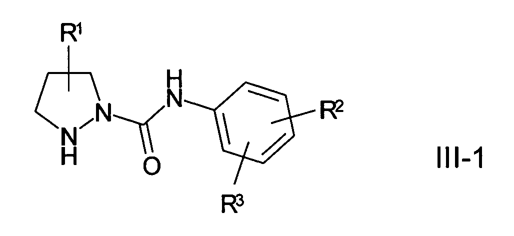 dans laquelle
R¹, R² et R³ ont la signification indiquée selon la revendication 1,
et, si R¹ désigne OH, le groupement OH est éventuellement sous forme protégée,
puis, si on le désire, le groupement protecteur d'OH est éliminé,
ou
b) un composé de formule IV 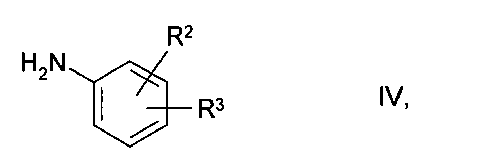
dans laquelle R² et R³ ont la signification indiquée selon la revendication 1,
est réagi avec un dérivé de chloroformiate pour donner un dérivé de carbamate intermédiaire,
qui est ensuite réagi avec un composé de formule III-2 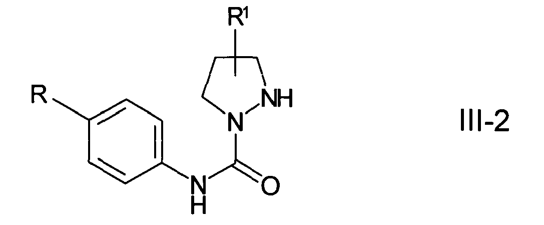 dans laquelle R et R¹ ont la signification indiquée selon la revendication 1,
et, si R¹ désigne OH, le groupement OH est éventuellement sous forme protégée,
puis, si on le désire, le groupement protecteur d'OH est éliminé,
et/ou
une base ou un acide de formule I est converti en l'un de ses sels.

4. Médicaments comprenant au moins un composé de formule I selon la revendication 1 ou 2 et/ou des dérivés, des sels, des solvats et des stéréoisomères pharmaceutiquement utilisables de ceux-ci, y compris des mélanges de ceux-ci selon des rapports quelconques, et éventuellement des excipients et/ou des adjuvants.

5. Médicaments comprenant au moins un composé de formule I selon la revendication 1 ou 2 et/ou des dérivés, des sels, des solvats et des stéréoisomères pharmaceutiquement utilisables de ceux-ci, y compris des mélanges de ceux-ci selon des rapports quelconques, et au moins un autre ingrédient actif médicamenteux.

6. Utilisation de composés selon la revendication 1 ou 2 et/ou de sels et de solvats physiologiquement acceptables de ceux-ci pour la préparation d'un médicament destiné au traitement de thromboses, de l'infarctus du myocarde, de l'artériosclérose, des inflammations, de l'apoplexie, de l'angine de poitrine, de la resténose après une angioplastie, de la boiterie intermittente, de la migraine, des tumeurs, des maladies tumorales et/ou des métastases tumorales.

7. Ensemble (kit) constitué de paquets distincts
(a) d'une quantité efficace d'un composé de formule I selon la revendication 1 ou 2 et/ou de dérivés, de sels, de solvats et de stéréoisomères pharmaceutiquement utilisables de ceux-ci, y compris des mélanges de ceux-ci selon des rapports quelconques,
et
(b) d'une quantité efficace d'un autre ingrédient actif médicamenteux.

8. Utilisation de composés de formule I selon la revendication 1 ou 2 et/ou de dérivés, de sels, de solvats et de stéréoisomères pharmaceutiquement utilisables de ceux-ci, y compris de mélanges de ceux-ci selon des rapports quelconques,
pour la préparation d'un médicament destiné au traitement de thromboses, de l'infarctus du myocarde, de l'artériosclérose, des inflammations, de l'apoplexie, de l'angine de poitrine, de la resténose après une angioplastie, de la boiterie intermittente, de la migraine, des tumeurs, des maladies tumorales et/ou des métastases tumorales, en association avec au moins un autre ingrédient actif médicamenteux.

9. Composés intermédiaires de formule III-1 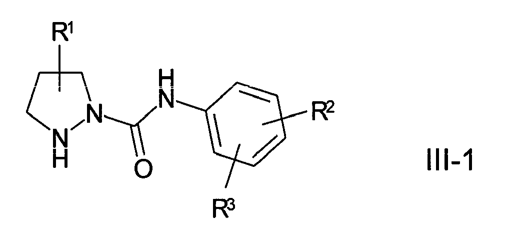 dans laquelle
R¹ désigne H, =O, OR⁶, OA, A-COO-, Ph-(CH₂)ₙ-COO- ou cycloalkyl-(CH₂)ₙ-COO-,
Ph désigne phényle non substitué,
R² désigne H, Hal ou A,
R³ désigne 2-oxopipéridin-1-yle, 2-oxopyrrolidin-1-yle, 2-oxo-1*H*-pyridin-1-yle, 3-oxomorpholin-4-yle, 4-oxo-1*H-*pyridin-1-yle, 2-oxo-1*H*-pyrazin-1-yle, 2-oxoimidazolidin-1-yle, 2,6-dioxopipéridin-1-yle, 2-oxopipérazin-1-yle, 2,6-dioxopipérazin-1-yle, 2,5-dioxopyrrolidin-1-yle, 2-oxo-1,3-oxazolidin-3-yle, 3-oxo-2*H*-pyridazin-2-yle, 2-caprolactam-1-yle (= 2-oxoazépan-1-yle), 2-aza-bicyclo[2.2.2]-octan-3-on-2-yle, 5,6-dihydro-1*H*-pyrimidin-2-oxo-1-yle, 2-oxo-1,3-oxazinan-3-yle ou 4*H*-1,4-oxazin-4-yle,
R⁶ désigne un groupement protecteur d'OH,
A désigne alkyle non ramifié, ramifié ou cyclique ayant 1-10 atomes de C, dans lequel 1-7 atomes de H peuvent également être remplacés par F et/ou chlore,
Hal désigne F, Cl, Br ou I,
n vaut 0, 1, 2, 3 ou 4,
et les isomères et les sels de ceux-ci.

10. Composés intermédiaires selon la revendication 9,
dans lesquels
R¹ désigne H, =O ou OR⁶,
R² désigne H, Hal ou A,
R³ désigne 2-oxopipéridin-1-yle, 2-oxopyrrolidin-1-yle, 2-oxo-1*H*-pyridin-1-yle, 3-oxomorpholin-4-yle, 4-oxo-1*H-*pyridin-1-yle, 2-oxo-1*H*-pyrazin-1-yle, 2-oxoimidazolidin-1-yle, 2,6-dioxopipéridin-1-yle, 2-oxopipérazin-1-yle, 2,6-dioxopipérazin-1-yle, 2,5-dioxopyrrolidin-1-yle, 2-oxo-1,3-oxazolidin-3-yle, 3-oxo-2*H*-pyridazin-2-yle, 2-caprolactam-1-yle (= 2-oxoazépan-1-yle), 2-aza-bicyclo[2.2.2]-octan-3-on-2-yle, 5,6-dihydro-1*H*-pyrimidin-2-oxo-1-yle, 2-oxo-1,3-oxazinan-3-yle, 4*H*-1,4-oxazin-4-yle,
R⁶ désigne un groupement protecteur alkylsilyle,
A désigne alkyle non ramifié, ramifié ou cyclique ayant 1-10 atomes de C, dans lequel 1-7 atomes de H peuvent également être remplacés par F et/ou chlore,
Hal désigne F, Cl, Br ou I,
n vaut 0, 1, 2, 3 ou 4,
et les isomères et les sels de ceux-ci.

11. Composés intermédiaires de formule III-2 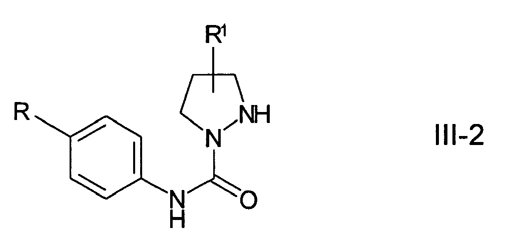 dans laquelle
R désigne Hal ou -C≡C-H,
R¹ désigne H, =O ou OR⁶,
R⁶ désigne un groupement protecteur alkylsilyle,
Hal désigne F, Cl, Br ou I,
où, si R¹ désigne H, R ne désigne pas Cl,
et les isomères et les sels de ceux-ci.

12. Composés intermédiaires de formule VI 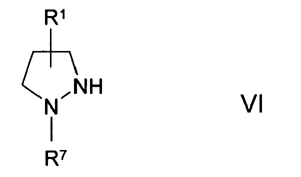 dans laquelle
R¹ désigne OH ou OR⁶,
R⁶ désigne un groupement protecteur silyle,
R⁷ désigne *tertio*-butyloxycarbonyle (BOC) ou benzyloxycarbonyle (Z),
et les isomères de ceux-ci.

13. Procédé de préparation de composés de formule VI 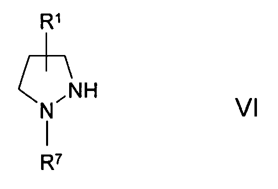 dans laquelle
R¹ désigne OH ou OR⁶,
R⁶ désigne un groupement protecteur silyle,
R⁷ désigne *tertio*-butyloxycarbonyle (BOC) ou benzyloxycarbonyle (Z),
et les isomères de ceux-ci,
pouvant être obtenus par la réaction d'un composé de formule VII
R⁷-NHNH₂ VII,
dans laquelle R⁷ désigne BOC ou Z,
avec du 1,3-dibromopropan-2-ol protégé par silyle, puis l'élimination éventuelle du groupement protecteur.
